# EUROPEAN PATENT APPLICATION

(11) **EP 4 545 532 A1**
(43) Date of publication of application: **30.04.2025**
(21) Application number: 23205559.0
(22) Date of filing: 24.10.2023
(51) Int. Cl.: C07D 487/08, C07D 213/18, C01B 25/10, C01B 35/06, C07B 39/00, C07C 17/10, C07C 17/14, C07C 45/63, C07C 67/287, C07C 67/307, C07C 211/05, C07C 211/46, C07C 231/12, C07C 253/30, C07D 213/74, C07D 233/58, C07D 307/92, C07J 9/00

(54) **ADDITIVES FOR IMPROVED C-H FLUORINATION REACTIONS**

(71) Applicant: Universität Regensburg, 93053 Regensburg (DE)
(72) Inventor: The designation of the inventor has not yet been filed
(74) Representative: Engelhard, Markus

(57) **Abstract**

The present invention relates to the use of 1-(chloromethyl)-1,4-diazabicyclo[2.2.2]octane-1,4-diium ditetrafluoroborate (SF-H) as additive in a fluorination reaction of a compound with a fluorination agent, such as 1-(chloromethyl)-4-fluoro-1,4-diazabicyclo[2.2.2]octane-1,4-diium ditetrafluoroborate (SF). The present invention further relates to the use of a hydrogen bond donor compound as additive in a fluorination reaction with a fluorination agent. The present invention also relates to methods for the fluorination of a compound with a fluorinating agent comprising the step of adding a hydrogen bond donor compound as an additive, such as SF-H. The present invention relates further to a method of synthesizing SF-H and to SF-H derivative compounds.

## Description

The present invention relates to the use of 1-(chloromethyl)-1,4-diazabicyclo[2.2.2]octane-1,4-diium ditetrafluoroborate (SF-H) as additive in a fluorination reaction of a compound with a fluorination agent, such as 1-(chloromethyl)-4-fluoro-1,4-diazabicyclo[2.2.2]octane-1,4-diium ditetrafluoroborate (SF). The present invention further relates to the use of a hydrogen bond donor compound as additive in a fluorination reaction with a fluorination agent. The present invention also relates to methods for the fluorination of a compound with a fluorinating agent comprising the step of adding a hydrogen bond donor compound as an additive, such as SF-H. The present invention relates further to a method of synthesizing SF-H and to SF-H derivative compounds.

### BACKGROUND OF THE INVENTION

Over the past decades, fluorination reactions have become a crucial aspect in contemporary synthesis of pharmaceuticals. The importance of organofluorine compounds to all areas of chemistry has increased exponentially over the past decades, including organic synthesis (see e.g. Lantaño *et al.,* 2017; Liang *et al.,* 2013), pharmaceutical science (Mei *et al.,* 2019), and materials development (Maienfisch and Hall, 2004). Remarkably, about half of the so-called "blockbuster drugs" and 26% of top 200 small molecule drugs by retail sales in 2021 contain at least one fluorine atom in their structure, including drugs used to treat cancer, HIV, smallpox and malarial infections (Purser *et al.,* 2008; Mei *et al.,* 2019). Moreover, fluorine atoms have dramatic effects on the biological activity of agrochemicals, such as insecticides, herbicides, and fungicides (Theodoridis 2018; Bume *et al.,* 2018). C-H/C-F substitution in organic molecules influences practically all physical, pharmacokinetic, adsorption, metabolic stability, and excretion properties of the compounds (Müller *et al.,* 2007; Hagmann 2008).

Unlike many other traditional energy resources, light is inexpensive, noncontaminating, nontoxic, ample (or "limitless" in the case of sunlight) and a renewable source of energy for "green" and environmentally friendly chemical synthesis. The field of visible-light photocatalysis, over the last few decades, has proven itself as a versatile, "green", and highly effective strategy for inducing different organic transformations under extremely mild conditions without threatening reagents and conditions (Roth 1989; Albini and Fagnoni, 2004; Albini and Fagnoni, 2008). Consequently, among the distinct strategies that complement the traditional fluorination methods, photosensitized fluorination reactions have undoubtedly been one of the most successful. Photosensitized fluorinations are particularly attractive for their applicability in late-stage functionalization (LSF) of complex molecules, mild conditions and use of light as a sustainable source of energy (Yakubov and Barham, 2020). Recently, we reported such method where methyl 4-fluorobenzoate can be used both as a photosenzitizer and photosensitization auxiliary for fluorination reactions of unactivated C(sp³)-H groups, which was applied to a broad substrate scope of target molecules including complex molecules such as natural products, pharmaceuticals and their derivatives (Yakubov *et al.,* 2022). Other photocatalytic direct fluorinations of unactivated C(sp³)-H groups were reported by Chen and co-workers (Xia *et al.,* 2013; Xia *et al.,* 2014) and Tan and co-workers (Kee *et al.,* 2014; Kee *et al.,* 2017) where they employed acetophenone or anthraquinone (AQN) as catalytic photosensitizers. Lectka and co-workers reported C(sp³)-H fluorination methods where they used benzil as a photocatalyst and native functionality within the target molecules to direct regioselective fluorinations (Bloom *et al.,* 2014-1; Bloom *et al.,* 2014-2). Another example of photosensitized C(sp³)-H fluorination is reported by Egami *et al.* (2018) where they found molecules with *N*-alkylphthalimides can undergo directed photochemical C(sp³)-H fluorination of their alkyl chains. As an alternative, thermal direct C(sp³)-H fluorination reactions that involve radical precursors were also reported. For example, Hua *et al.* (2017) and Amaoka *et al.* (2013) report direct fluorination reactions where they employed glycine or *N,N-*dihydroxypyromellitimide as radical precursors.

The biggest challenges that still remain present in the fluorination methods known as of yet is the low or variable yields of the fluorinated products, the long reaction times and the oftentimes need for strictly inert conditions (such as glovebox preparations). In this context, procedures for the construction of carbon-fluorine bonds are highly prized because of the scarcity of methods and the value of the products across applied chemistry.

There is a need in the art for improved means for fluorination methods, which in particular increase the yield and/or decrease the reaction times.

### SUMMARY OF THE INVENTION

According to the present invention this object is solved by using 1-(chloromethyl)-1,4-diazabicyclo[2.2.2]octane-1,4-diium ditetrafluoroborate (SF-H) as additive in a C-H fluorination reaction of a compound with a fluorination agent.

According to the present invention this object is solved by using a hydrogen bond donor compound as additive in a C-H fluorination reaction of a compound with a fluorination agent.

According to the present invention this object is solved by a method for the C-H fluorination of a compound with a fluorinating agent, comprising the step:
adding 1-(chloromethyl)-1,4-diazabicyclo[2.2.2]octane-1,4-diium ditetrafluoroborate (SF-H) as additive.

According to the present invention this object is solved by a method for the C-H fluorination of a compound with a fluorination agent, comprising the step:
adding a hydrogen bond donor compound as additive.

According to the present invention this object is solved by a method of synthesizing 1-(chloromethyl)-1,4-diazabicyclo[2.2.2]octane-1,4-diium ditetrafluoroborate (SF-H), comprising the steps of
1) alkylation of 1,4-diazabicyclo[2.2.2]octane in dichloromethane;
2) protonation and ion exchange via addition of tetrafluoroboric acid to the mixture of step 1) and obtaining SF-H;
   or
2') protonation via addition of hydrochloric acid to the mixture of step 1) and obtaining 1-(chloromethyl)-1,4-diazabicyclo[2.2.2]octane-1,4-diium dichloride;
3') ion exchange via addition of a solution of sodium tetrafluoroborate (NaBF₄) to the product of step 2') and obtaining SF-H;
   or
2") ion exchange via addition of a solution of NaBF₄ to the product of step 1) and obtaining 1-(chloromethyl)-1,4-diazabicyclo[2.2.2]octan-1-ium tetrafluoroborate;
3") protonation and ion exchange via addition of tetrafluoroboric acid to the product of step 2") and obtaining SF-H.

According to the present invention this object is solved by providing a compound having the formula V or VI 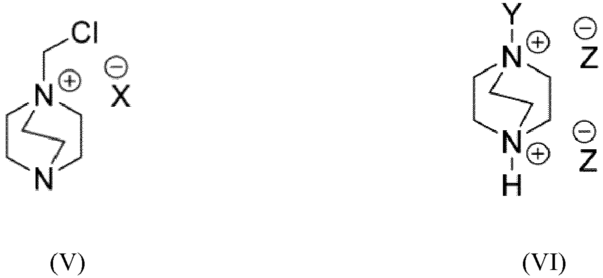 wherein
X is Cl or BF₄,
Y is CH₂Cl or CH₃, and
Z is Cl, BF₄ or PF₆.

According to the present invention this object is solved by providing a compound selected from

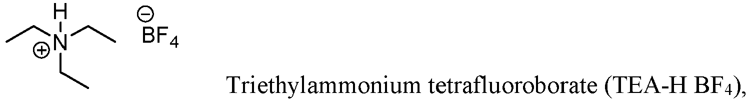

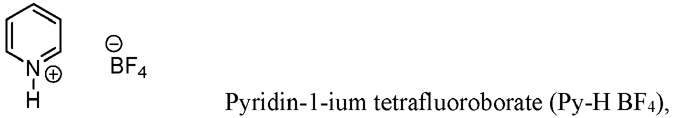

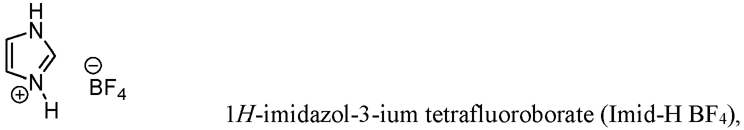

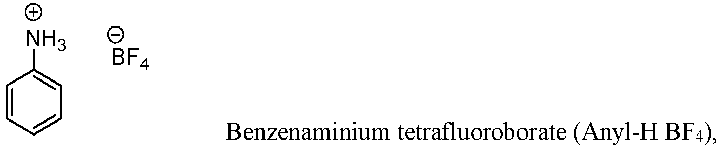

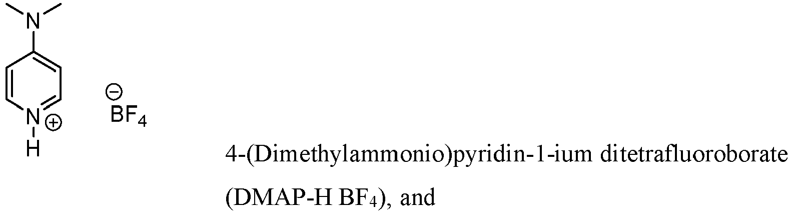

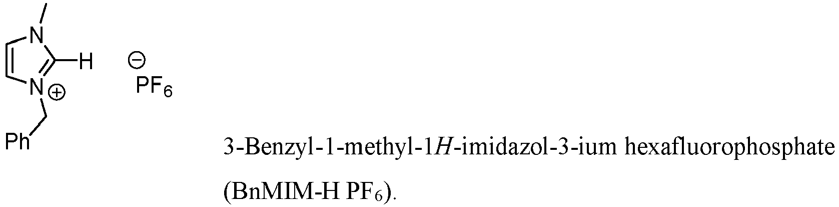

According to the present invention this object is solved by using a compound of the present invention as additive in a C-H fluorination reaction of a compound with a fluorination agent.

According to the present invention this object is solved by using a hydrogen bond donor compound as additive in a C-H fluorination reaction of a compound with a fluorination agent, wherein the fluorination agent is preferably an organic fluorination agent which is more preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetraetrafluoroborate) (SF II) and *N*-Fluorobenzenesulfonimide (NFSI),
wherein the hydrogen bond donor compound is preferably a compound of the present invention.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 20" should be interpreted to include not only the explicitly recited values of 1 to 20, but also include individual values and sub-ranges within the indicated range. Thus, included in this numerical range are individual values such as 1, 2, 3, 4, 5 .... 17, 18, 19, 20 and sub-ranges such as from 2 to 10, 8 to 15, etc. This same principle applies to ranges reciting only one numerical value, such as "higher than 100". Furthermore, such an interpretation should apply regardless of the breadth of the range or the characteristics being described.

### Additives for C-H fluorination reactions

As outlined above, the present invention provides 1-(chloromethyl)-1,4-diazabicyclo[2.2.2]octane-1,4-diium ditetrafluoroborate (SF-H) as additive in a C-H fluorination reaction of a compound with a fluorination agent.

Preferably, the fluorination agent is an organic fluorination agent.

In one embodiment, the organic fluorination agent is preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetrafluoroborate) (SF II) and *N-*Fluorobenzenesulfonimide (NFSI).

In one embodiment, the fluorination agent is an electrophilic fluorination agent.

An "electrophilic fluorination agent" is an electrophilic source of fluorine, where the electron-deficient fluorine atom is the active species of the reaction that serves as a site of nucleophilic attack by a substrate behaving as a nucleophile.

As outlined above, the present invention provides a hydrogen bond donor compound as additive in a C-H fluorination reaction of a compound with a fluorination agent.

An "additive" as used herein is a compound which is added to a reaction, in this case a C-H fluorination reaction of a compound with a fluorination agent. The additive is not the fluorination agent, it is an exogenous compound that need not contain fluorine atoms. The additives of the present invention are preferably performance enhancing additives.

Preferably, the fluorination agent is an organic fluorination agent.

In one embodiment, the organic fluorination agent is preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetrafluoroborate) (SF II) and N-Fluorobenzenesulfonimide (NFSI).

Preferably, the hydrogen bond donor compound is a Brønsted acid.

In one embodiment, the hydrogen bond donor compound is a Bronsted acid having a pKa of less than about 15 in water or DMSO.

In one embodiment, the hydrogen bond donor compound is a Bronsted acid selected from an organic acid having a formula selected from formula I, II, III, IVa or IVb.

An organic acid having formula I: 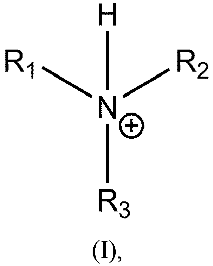 is an organic acid wherein:
R¹, R² and R³ are each independently selected from
   H,
   an unsubstituted or substituted aliphatic carbon chain (such as C₁-C₆ alkyl, C₁-C₆ alkenyl, C₁-C₆ alkynyl),
   C₃-C₁₀ heterocyclyl,
   unsubstituted or substituted aromatic carbon chain (such as C₃-C₁₀ aryl), and halogen, and
can be the same or different.

Preferably, not all of R¹, R² and R³ are H.

An organic acid having formula II: 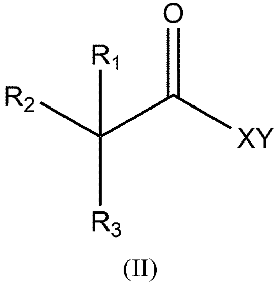 is an organic acid wherein:
R¹, R² and R³ are each independently selected from
   H,
   an unsubstituted or substituted aliphatic carbon chain (such as C₁-C₆ alkyl, C₁-C₆ alkenyl, C₁-C₆ alkynyl),
   C₃-C₁₀ heterocyclyl,
   unsubstituted or substituted aromatic carbon chain (such as C₃-C₁₀ aryl), and halogen, and
   can be the same or different,
X is S or O,
Y is H(¹H) or ²H.

In one embodiment, R¹, R² and R³ are each independently selected from H and C₁-C₆ alkyl, more preferably H and methyl.

An organic acid having formula III: 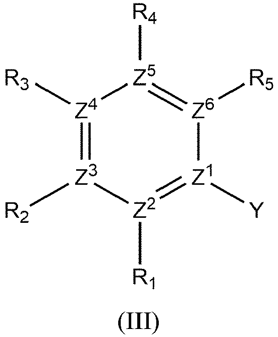 is an organic acid wherein:
R¹ to R⁵ are each independently selected from
   H,
   an unsubstituted or substituted aliphatic carbon chain (such as C₁-C₆ alkyl, C₁-C₆ alkenyl, C₁-C₆ alkynyl),
   C₃-C₁₀ heterocyclyl,
   unsubstituted or substituted aromatic carbon chain (such as C₃-C₁₀ aryl), and
   -NR⁶,
   halogen, and
   can be the same or different, and,
R⁶ is H or C₁-C₆ alkyl,
Y is H(¹H) or ²H,
Z¹ to Z⁶ are each independently selected from C, N, O and S,
wherein not all of Z¹ to Z⁶ are C, or
wherein not all of Z¹ to Z⁶ are N, or
wherein not all of Z¹ to Z⁶ are O or
wherein not all of Z¹ to Z⁶ are S.

In one embodiment, Z¹ is N and Z² to Z⁶ are C. In this embodiment, the organic acid has formula III-1 (pyridine/pyridinium scaffold):

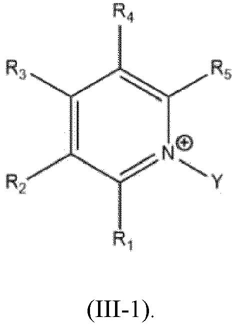

In one embodiment, Z¹ and Z⁴ are N and Z², Z³, Z⁵ and Z⁶ are C (pyrazine scaffold).

In one embodiment, Z¹ and Z³ are N and Z², Z⁴, Z⁵ and Z⁶ are C (pyrimidine scaffold).

In one embodiment, R³ is -NR⁶.

In one embodiment, R¹ to R⁵ are each independently selected from H and C₁-C₆ alkyl, more preferably H and methyl.

An organic acid having formula IVa or IVb: 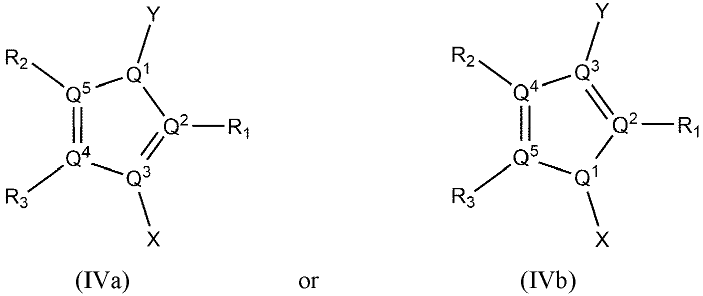 is an organic acid wherein:
R¹, R² and R³ are each independently selected from
   H,
   an unsubstituted or substituted aliphatic carbon chain (such as C₁-C₆ alkyl, C₁-C₆ alkenyl, C₁-C₆ alkynyl),
   C₃-C₁₀ heterocyclyl,
   unsubstituted or substituted aromatic carbon chain (such as C₃-C₁₀ aryl), and halogen, and
   can be the same or different,
X is selected from H(¹H), ²H or R⁴,
Y is selected from H(¹H), ²H or R⁴,
R⁴ is selected from an unsubstituted or substituted aliphatic carbon chain (such as C₁-C₆ alkyl, C₁-C₆ alkenyl, C₁-C₆ alkynyl), C₃-C₁₀ heterocyclyl, unsubstituted or substituted aromatic carbon chain (such as C₃-C₁₀ aryl), and halogen,
Q¹ to Q⁵ are each independently selected from C, N, O and S,
wherein not all of Q¹ to Q⁵ are C, or
wherein not all of Q¹ to Q⁵ are N, or
wherein not all of Q¹ to Q⁵ are O or
wherein not all of Q¹ to Q⁵ are S.

In one embodiment, R¹, R² and R³ are each independently selected from H and C₁-C₆ alkyl, more preferably H and methyl.

In one embodiment, Q¹ and Q³ are N and Q², Q⁴ and Q⁵ are C. In this embodiment, the organic acid has formula IVa-1 or IVb-1 (imidazole/imidazolium scaffold):

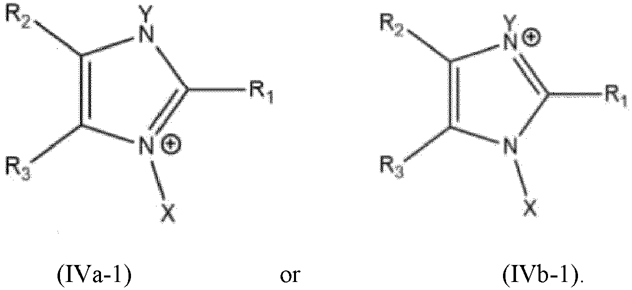

In one embodiment, Q¹ and Q² are N and Q³, Q⁴ and Q⁵ are C (pyrazole scaffold).

In one embodiment, Q¹, Q² and Q³ are N and Q⁴ and Q⁵ are C (triazole scaffold).

In one embodiment, Q¹ to Q⁴ are N and Q⁵ is C (tetrazole scaffold).

In one embodiment, Q¹ is N, Q³ is O and Q², Q⁴ and Q⁵ are C (oxazole scaffold).

In one embodiment, Q¹ is N, Q³ is S and Q², Q⁴ and Q⁵ are C (thiazole scaffold).

An organic acid having a formula selected from formula I, II, III, IVa or IVb, as defined above, preferably comprises an anion.

Typical anions are F⁻, Cl⁻, Br⁻, I⁻, PF₆⁻. BF₄⁻, ClO₄⁻, CO₃²⁻ (carbonate), SO₄²⁻ (sulfate). Further anions are known to the skilled artisan.

See also https://sciencenotes.org/common-anions-list-and-formulas/.

In one embodiment, the hydrogen bond donor compound is 1-(chloromethyl)-1,4-diazabicyclo[2.2.2]octane-1,4-diium ditetrafluoroborate (SF-H).

Preferably, the C-H fluorination reaction comprises or is a fluorination of unactivated C(sp³)-H group(s) of a compound.

In one embodiment the fluorination reaction is an electrophilic fluorination reaction.

In one embodiment the fluorination reaction is a photochemical fluorination reaction.

A photochemical fluorination reaction is any fluorination reaction that involves the absorption of energy by a molecular entity (catalyst or target substrate) in the form of light. The wavelengths of light range from the UV to the visible spectrum, namely about 200 nm to about 1200 nm.

In one embodiment the fluorination reaction is a thermal fluorination reaction.

A thermal fluorination reaction is any fluorination reaction in which the reaction partners or catalyst harvest energy in the form of heat.

In one embodiment the fluorination reaction is a photocatalytic fluorination reaction.

A photocatalytic fluorination reaction is any photochemical fluorination reaction that involves a molecular entity that absorbs light and that is regenerated after the chemical reaction (serves as a catalyst). The catalyst can engage the target substrate either by transferring energy or by transferring electrons or electron-holes.

In one embodiment the fluorination reaction is a photosensitized fluorination reaction.

A photosensitized fluorination reaction is any photochemical fluorination reaction that involves a photosensitizer (a substance capable of absorbing light and transferring the energy to the desired target molecule or functional group). The photosensitizer can be employed either in a catalytic fashion (i.e. is regenerated) or a stoichiometric fashion (i.e. is not regenerated), and can also be a functional group built in to the target molecule to be fluorinated.

In one embodiment the fluorination reaction is an electrophilic, a photochemical, a thermal, a photocatalytic and/or a photosensitized fluorination reaction.

In one embodiment, the hydrogen bond donor compound is used in a ratio of about 1:20 to about 20:1 of the fluorination agent, preferably about 1:10 to about 10:1, wherein the fluorination agent is preferably SF.

In one embodiment, SF-H is used in the range of about 0.1 eq to about 10 eq of the fluorination agent, which is preferably SF.

In a preferred embodiment, the hydrogen bond donor compound is used in excess of the fluorination agent, which is preferably SF.

For example, the excess of hydrogen bond donor compound is in the range from about 10 to about 1.5.

For example, where SF-H is used as the hydrogen bond donor compound: 10 eq SF-H are used. For example, 6 eq SF-H are used. For example, 2 eq SF-H are used.

In one preferred embodiment, the ratio of hydrogen bond donor compound, preferably SF-H, to fluorination agent, preferably SF, is about 2: 1.

The compounds to be fluorinated or the fluorinated products are preferably bioactive molecules, such as pharmaceuticals and/or agrochemicals.

For example, pharmaceuticals can be
anti-inflammatory compounds,
   such as fluticasone propionate (as a fluorinated product),
anti-viral compounds,
   such as sofosbuvir (HCV infections) (as a fluorinated product),
steroids,
peptides.

For example, agrochemicals can be
herbicides,
   such as fluroxypyr methylheptyl (as a fluorinated product),
insecticides,
   such as flufenoxuron (as a fluorinated product).

In one embodiment, the fluorinated compounds can be obtained in gram scale.

In one embodiment, the hydrogen bond donor compound, preferably SF-H, is reusable.

### Fluorination reactions with additives

As outlined above, the present invention provides a method for the C-H fluorination of a compound with a fluorinating agent.

Said method comprises the step:
adding 1-(chloromethyl)-1,4-diazabicyclo[2.2.2]octane-1,4-diium ditetrafluoroborate (SF-H) as additive.

Preferably, the fluorination agent is an organic fluorination agent.

In one embodiment, the organic fluorination agent is preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetrafluoroborate) (SF II) and *N-*Fluorobenzenesulfonimide (NFSI).

As outlined above, the present invention provides a method for the C-H fluorination of a compound with a fluorination agent.

Said method comprises the step:
adding a hydrogen bond donor compound as additive,

Preferably, the fluorination agent is an organic fluorination agent.

In one embodiment, the organic fluorination agent is preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetrafluoroborate) (SF II) and *N-*Fluorobenzenesulfonimide (NFSI).

Preferably and as discussed above, the hydrogen bond donor compound is a Bronsted acid.

In one embodiment, the hydrogen bond donor compound is a Bronsted acid having a pKa of less than about 15 in water or DMSO.

In one embodiment, the hydrogen bond donor compound is a Bronsted acid selected from an organic acid having a formula selected from formula I, II, III, IVa or IVb.

As discussed above, an organic acid having formula I: 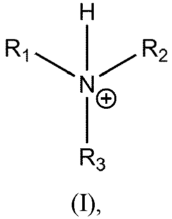 is an organic acid wherein:
R¹, R² and R³ are each independently selected from
   H,
   an unsubstituted or substituted aliphatic carbon chain (such as C₁-C₆ alkyl, C₁-C₆ alkenyl, C₁-C₆ alkynyl),
   C₃-C₁₀ heterocyclyl,
   unsubstituted or substituted aromatic carbon chain (such as C₃-C₁₀ aryl), and halogen, and
can be the same or different, wherein not all of R¹, R² and R³ are H.

As discussed above, an organic acid having formula II: 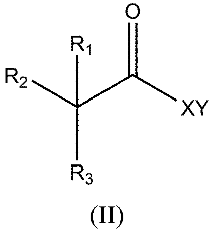 is an organic acid wherein:
R¹, R² and R³ are each independently selected from
   H,
   an unsubstituted or substituted aliphatic carbon chain (such as C₁-C₆ alkyl, C₁-C₆ alkenyl, C₁-C₆ alkynyl),
   C₃-C₁₀ heterocyclyl,
   unsubstituted or substituted aromatic carbon chain (such as C₃-C₁₀ aryl), and halogen, and
   can be the same or different,
X is S or O,
Y is H(¹H) or ²H.

In one embodiment, R¹, R² and R³ are each independently selected from H and C₁-C₆ alkyl, more preferably H and methyl.

As discussed above, an organic acid having formula III: 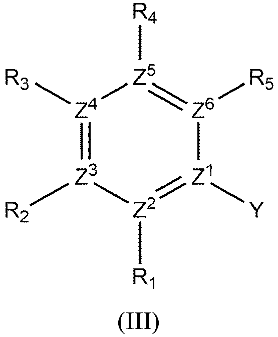 is an organic acid wherein:
R¹ to R⁵ are each independently selected from
   H,
   an unsubstituted or substituted aliphatic carbon chain (such as C₁-C₆ alkyl, C₁-C₆ alkenyl, C₁-C₆ alkynyl),
   C₃-C₁₀ heterocyclyl,
   unsubstituted or substituted aromatic carbon chain (such as C₃-C₁₀ aryl), and
   -NR⁶,
   halogen, and
   can be the same or different, and,
R⁶ is H or C₁-C₆ alkyl,
Y is H(¹H) or ²H,
Z¹ to Z⁶ are each independently selected from C, N, O and S,
wherein not all of Z¹ to Z⁶ are C, or
wherein not all of Z¹ to Z⁶ are N, or
wherein not all of Z¹ to Z⁶ are O or
wherein not all of Z¹ to Z⁶ are S.

In one embodiment, Z¹ is N and Z² to Z⁶ are C. In this embodiment, the organic acid has formula III-1 (pyridine/pyridinium scaffold):

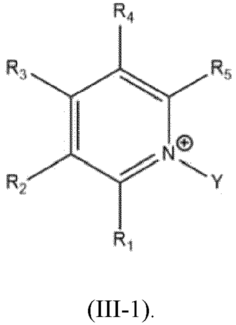

In one embodiment, Z¹ and Z⁴ are N and Z², Z³, Z⁵ and Z⁶ are C (pyrazine scaffold).

In one embodiment, Z¹ and Z³ are N and Z², Z⁴, Z⁵ and Z⁶ are C (pyrimidine scaffold).

In one embodiment, R³ is -NR⁶.

In one embodiment, R¹ to R⁵ are each independently selected from H and C₁-C₆ alkyl, more preferably H and methyl.

As discussed above, an organic acid having formula IVa or IVb: 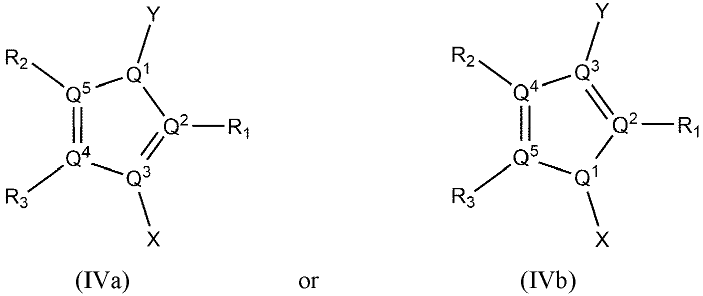 is an organic acid wherein:
R¹, R² and R³ are each independently selected from
   H,
   an unsubstituted or substituted aliphatic carbon chain (such as C₁-C₆ alkyl, C₁-C₆ alkenyl, C₁-C₆ alkynyl),
   C₃-C₁₀ heterocyclyl,
   unsubstituted or substituted aromatic carbon chain (such as C₃-C₁₀ aryl), and halogen, and
   can be the same or different,
X is selected from H(¹H), ²H or R⁴,
Y is selected from H(¹H), ²H or R⁴,
R⁴ is selected from an unsubstituted or substituted aliphatic carbon chain (such as C₁-C₆ alkyl, C₁-C₆ alkenyl, C₁-C₆ alkynyl), C₃-C₁₀ heterocyclyl, unsubstituted or substituted aromatic carbon chain (such as C₃-C₁₀ aryl), and halogen,
Q¹ to Q⁵ are each independently selected from C, N, O and S,
wherein not all of Q¹ to Q⁵ are C, or
wherein not all of Q¹ to Q⁵ are N, or
wherein not all of Q¹ to Q⁵ are O or
wherein not all of Q¹ to Q⁵ are S.

In one embodiment, R¹, R² and R³ are each independently selected from H and C₁-C₆ alkyl, more preferably H and methyl.

In one embodiment, Q¹ and Q³ are N and Q², Q⁴ and Q⁵ are C. In this embodiment, the organic acid has formula IVa-1 or IVb-1 (imidazole/imidazolium scaffold):

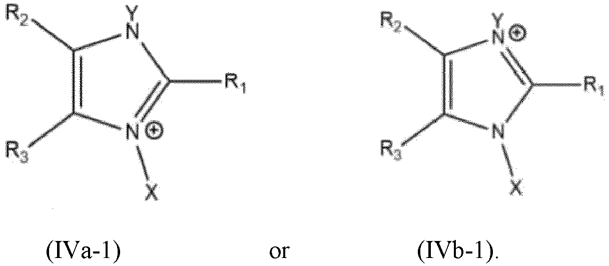

In one embodiment, Q¹ and Q² are N and Q³, Q⁴ and Q⁵ are C (pyrazole scaffold).

In one embodiment, Q¹, Q² and Q³ are N and Q⁴ and Q⁵ are C (triazole scaffold).

In one embodiment, Q¹ to Q⁴ are N and Q⁵ is C (tetrazole scaffold).

In one embodiment, Q¹ is N, Q³ is O and Q², Q⁴ and Q⁵ are C (oxazole scaffold).

In one embodiment, Q¹ is N, Q³ is S and Q², Q⁴ and Q⁵ are C (thiazole scaffold).

An organic acid having a formula selected from formula I, II, III, IVa or IVb, as defined above, preferably comprises an anion.

Typical anions are F⁻, Cl⁻, Br⁻, I⁻, PF₆⁻. BF₄⁻, ClO₄⁻, CO₃²⁻ (carbonate), SO₄²⁻ (sulfate). Further anions are known to the skilled artisan.

See also https://sciencenotes.org/common-anions-list-and-formulas/.

In one embodiment, the hydrogen bond donor compound is 1-(chloromethyl)-1,4-diazabicyclo[2.2.2]octane-1,4-diium ditetrafluoroborate (SF-H).

In one embodiment and as discussed above, the fluorination reaction is an electrophilic fluorination reaction.

In one embodiment and as discussed above, the fluorination reaction is a photochemical fluorination reaction.

In one embodiment and as discussed above, the fluorination reaction is a thermal fluorination reaction.

In one embodiment and as discussed above, the fluorination reaction is a photocatalytic fluorination reaction.

In one embodiment and as discussed above, the fluorination reaction is a photosensitized fluorination reaction.

In one embodiment and as discussed above, the fluorination reaction is an electrophilic, a photochemical, a thermal, a photocatalytic and/or a photosensitized fluorination reaction.

In one embodiment, the hydrogen bond donor compound is used in a ratio of about 1:20 to about 20:1 of the fluorination agent, preferably about 1:10 to about 10:1, wherein the fluorination agent is preferably SF.

In one embodiment, SF-H is used in the range of about 0.1 eq to about 10 eq of the fluorination agent, which is preferably SF.

In a preferred embodiment, the hydrogen bond donor compound is used in excess of the fluorination agent, which is preferably SF.

For example, the excess of of hydrogen bond donor compound is in the range from about 10 to about 1.5.

For example, where SF-H is used as the hydrogen bond donor compound: 10 eq SF-H are used. For example, 6 eq SF-H are used. For example, 2 eq SF-H are used.

In one preferred embodiment, the ratio of hydrogen bond donor compound, preferably SF-H, to fluorination agent, preferably SF, is about 2:1.

The compounds to be fluorinated are preferably bioactive molecules, such as pharmaceuticals and/or agrochemicals.

For example, pharmaceuticals can be
anti-inflammatory compounds, such as fluticasone propionate,
anti-viral compounds, such as sofosbuvir (HCV infections),
steroids,
peptides.

For example, agrochemicals can be
herbicides, such as fluroxypyr methylheptyl,
insecticides, such as flufenoxuron.

In one embodiment, the fluorinated compounds can be obtained in gram scale.

In one embodiment, the hydrogen bond donor compound, preferably SF-H, is reusable.

### Synthesis of SF-H

As outlined above, the present invention provides a method of synthesizing 1-(chloromethyl)-1,4-diazabicyclo[2.2.2]octane-1,4-diium ditetrafluoroborate (SF-H).

The method comprises an *alkylation* step as step 1):
1) alkylation of 1,4-diazabicyclo[2.2.2]octane in dichloromethane (DABCO).

In Step 1), 1,4-diazabicyclo[2.2.2]octane in dichloromethane (DABCO), also known as triethylenediamine (TEDA), can be dissolved in DCM. It can be refluxed, such as under 40°C for 24 h.

The reaction mixture is further used in Step 2) or Step 2') or Step 2").

In one embodiment, the method comprises the *alkylation* step as step 1) followed by a *protonation*/*ion exchange* step. Said method comprises the steps of
1) alkylation of 1,4-diazabicyclo[2.2.2]octane in dichloromethane;
2) protonation and ion exchange via addition of tetrafluoroboric acid (HBF₄) to the mixture of step 1) and obtaining SF-H.

In Step 2) before HBF₄ (such as 3 eq HBF₄) is added, the reaction mixture of Step 1) can be concentrated and a dry product can be obtained. Said dry product is then dissolved in a solvent, such as acetonitrile or acetone.

In Step 2), after the addition of HBF₄, the reaction mixture can be stirred, such as for one hour. After the stirring, a white solid can precipitated and can be filtered and dried *in vacuo* to obtain the product SF-H.

In one embodiment, the method comprises the *alkylation* step as step 1) followed by a *protonation* step which is followed by an *ion exchange* step. Said method comprises the steps of
1) alkylation of 1,4-diazabicyclo[2.2.2]octane in dichloromethane;
2') protonation via addition of hydrochloric acid to the mixture of step 1) and obtaining 1-(chloromethyl)-1,4-diazabicyclo[2.2.2]octane-1,4-diium dichloride;
3') ion exchange via addition of a solution of sodium tetrafluoroborate (NaBF₄) to the product of step 2'), namely 1-(chloromethyl)-1,4-diazabicyclo[2.2.2]octane-1,4-diium dichloride, and obtaining SF-H.

In Step 2'), after addition of HCl (such as 2 eq HCl), the reaction mixture can be stirred, such as for one hour. After the stirring, it can be concentrated *in vacuo* and a solid can be obtained as the product of this step. The product can be a white solid. The product is 1-(chloromethyl)-1,4-diazabicyclo[2.2.2]octane-1,4-diium dichloride.

In Step 3'), after the addition of NaBF₄ (such as 2.1 eq NaBF₄) the reaction mixture can be stirred, such as for one hour. Sodium chloride can precipitate as a white solid that can be filtered out. The filtrate can be dried *in vacuo* and the product SF-H is obtained.

In one embodiment, the method comprises the *alkylation* step as step 1) followed by an *ion exchange* step which is followed by *a protonation*/*ion exchange* step. Said method comprises the steps of
1) alkylation of 1,4-diazabicyclo[2.2.2]octane in dichloromethane;
2") ion exchange via addition of a solution of NaBF₄ to the product of step 1) and obtaining 1-(chloromethyl)-1,4-diazabicyclo[2.2.2]octan-1-ium tetrafluoroborate;
3") protonation and ion exchange via addition of tetrafluoroboric acid to the product of step 2"), namely 1-(chloromethyl)-1,4-diazabicyclo[2.2.2]octan-1-ium tetrafluoroborate, and obtaining SF-H.

In Step 2"), after addition of NaBF₄, (such as 1.1 eq NaBF₄) the reaction mixture can be stirred, such as for 1 hour. After the stirring, it can be concentrated *in vacuo* and a solid can be obtained as the product of this step. The product can be a white solid. The product is 1-(chloromethyl)-1,4-diazabicyclo[2.2.2]octan-1-ium tetrafluoroborate.

In Step 3"), the product of step 2") can be dissolved in acetonitrile or acetone before the addition of the tetrafluoroboric acid (such as 1.5 eq HBF₄).

The reaction mixture can be stirred, such as for 1 hour. A white solid can be precipitated and can be filtered and can be dried *in vacuo* and the product SF-H is obtained

### SF-H derivatives

As outlined above, the present invention provides a compound having the formula V or VI 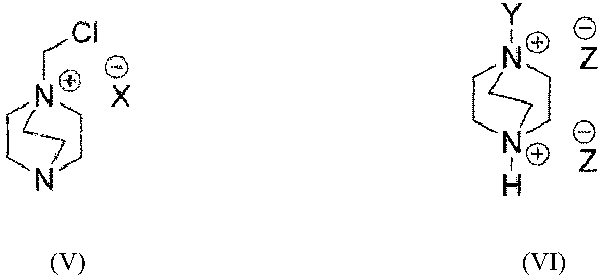 wherein
**X** is Cl or BF₄,
**Y** is CH₂Cl or CH₃, and
**Z** is Cl, BF₄ or PF₆.

In one embodiment, the compound has formula V and **X** is Cl.

In one embodiment, the compound has formula V and **X** is BF₄.

In one embodiment, the compound has formula VI and **Y** is CH₂Cl and **Z** is BF₄.

In one embodiment, the compound has formula VI and **Y** is CH₂Cl and **Z** is Cl.

In one embodiment, the compound has formula VI and **Y** is CH₂Cl and **Z** is PF₆.

In one embodiment, the compound has formula VI and **Y** is CH₃ and **Z** is BF₄.

In one embodiment, the compound has formula VI and **Y** is CH₃ and **Z** is Cl.

In one embodiment, the compound has formula VI and **Y** is CH₃ and **Z** is PF₆.

As outlined above, the present invention provides a compound selected from

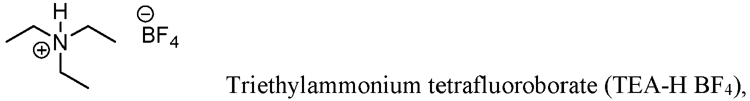

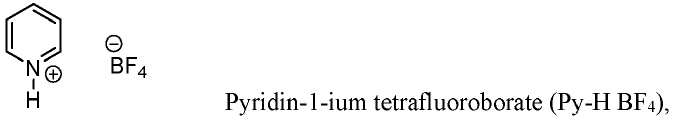

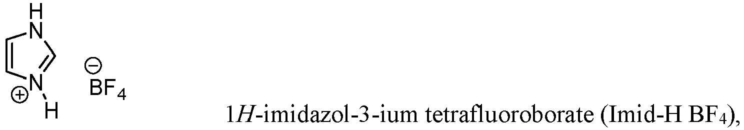

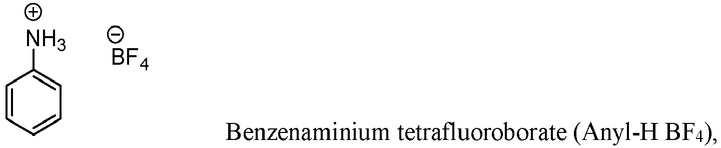

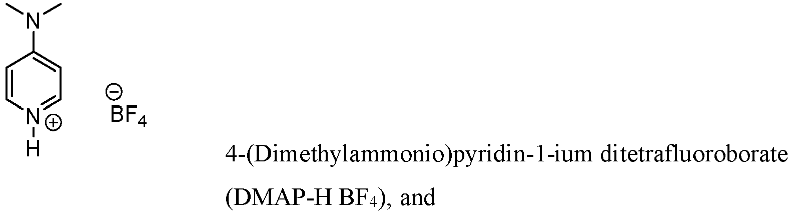

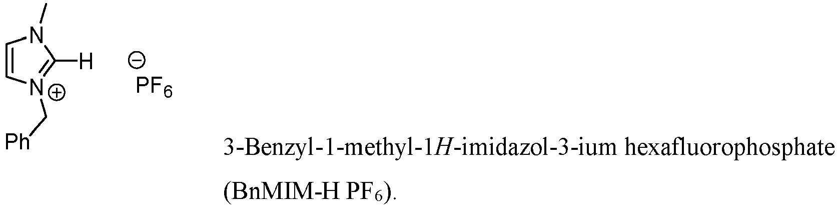

The compounds of formula V and VI can also be used as additives in the fluorination reactions, as described above.

The compounds TEA-H BF₄, Py-H BF₄, Imid-H BF₄, Anyl-H BF₄, DMAP-H BF₄, and BnMIM-H PF₆ can also be used as additives in the fluorination reactions, as described above

As discussed above, the present invention provides using a compound of formula V, a compound of formula VI, TEA-H BF₄, Py-H BF₄, Imid-H BF₄, Anyl-H BF₄, DMAP-H BF₄, or BnMIM-H PF₆ as additive in a C-H fluorination reaction of a compound with a fluorination agent.

The fluorination agent is preferably an organic fluorination agent, which is more preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetraetrafluoroborate) (SF II) and *N*-Fluorobenzenesulfonimide (NFSI).

As discussed above, the present invention provides using a hydrogen bond donor compound as additive in a C-H fluorination reaction of a compound with a fluorination agent.

The fluorination agent is preferably an organic fluorination agent, which is more preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetraetrafluoroborate) (SF II) and *N*-Fluorobenzenesulfonimide (NFSI).

The hydrogen bond donor compound is preferably a compound of formula V, a compound of formula VI, TEA-H BF₄, Py-H BF₄, Imid-H BF₄, Anyl-H BF₄, DMAP-H BF₄, or BnMIM-H PF₆.

In one embodiment, and as discussed above, the C-H fluorination reaction is an electrophilic, a photochemical, a thermal, a photocatalytic and/or a photosensitized fluorination reaction. In one embodiment, and as discussed above, the compounds to be fluorinated are bioactive molecules, such as pharmaceuticals and/or agrochemicals.

In one embodiment, and as discussed above, the fluorinated compounds can be obtained in gram scale.

### Further description of preferred embodiments

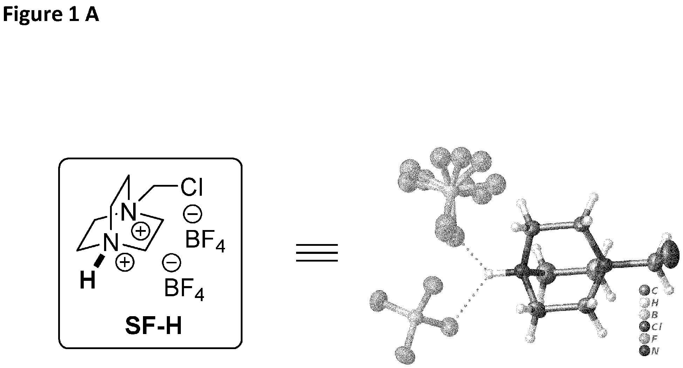

1-(Chloromethyl)-1,4-diazabicyclo[2.2.2]octane-1,4-diium ditetrafluoroborate (SF-H, see Figure 1A) is a byproduct of many fluorination reactions where 1-(chloromethyl)-4-fluoro-1,4-diazabicyclo[2.2.2]octane-1,4-diium ditetrafluoroborate (SF) is deployed as an electrophilic fluorine source. SF is commercially available under the name Selectfluor^{®}.

Herein, we report the discovery of SF-H as a novel, cheap, and recoverable additive that improves the yields of diverse fluorination methods. We further show several different examples of both thermal and photocatalytic fluorination studies where SF-H markedly increases the efficiency, rapidity, and practicality of C(sp³)-H fluorination reactions. Furthermore, we present non-catalytic SF-H - assisted fluorination method that delivers high yields in short reaction time.

At first, we used one of the substrates as a benchmark, that provided low yield of the fluorinated product under our reported method for photosensitized fluorinations (see Yakubov *et al.,* 2022) - 4-phenylbutyl benzoate (1b). Substrate 1b underwent fluorination with SF and 1 mol% photocatalyst (MFB) under 400 nm LED yielding only 10% fluorinated product (2b). When model substrate 1b was fluorinated in the presence of 2.0 eq. SF-H as additive, the yield of the product was increased from 10% to 66% (Scheme 1). Thus, it was encouraging to find that our methodology dramatically improves the efficiency of the reactions.

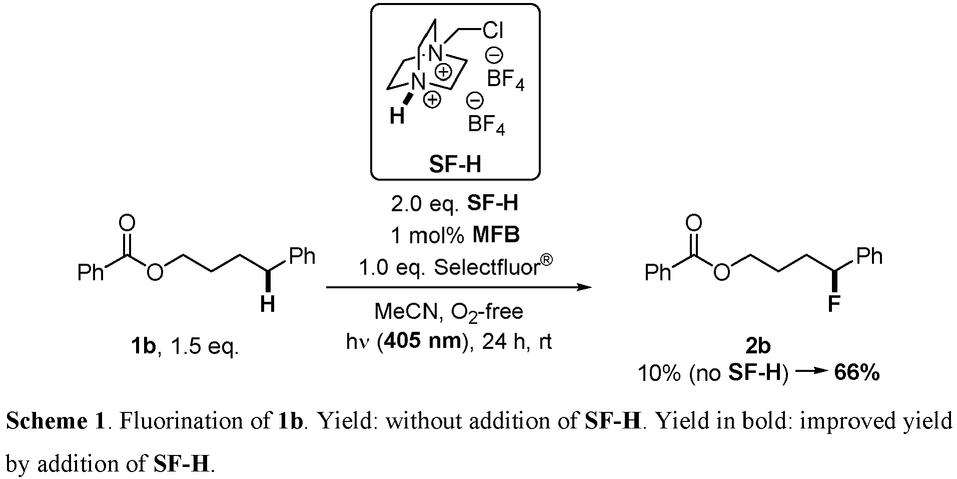

Next, we targeted the effect of SF-H on our reported method for photosensitized fluorinations (see Yakubov *et al.,* 2022). Specifically, we targeted substrate 1c, because when subjected to fluorination reaction with SF under 400 nm LED, it provided low yield (38%) of the fluorinated product. When the reaction was repeated with 2.0 eq. of SF-H as an additive, the yield increased from 38% to 65% (Scheme 2). Based on these results, we aimed to use SF-H in other fluorination methods.

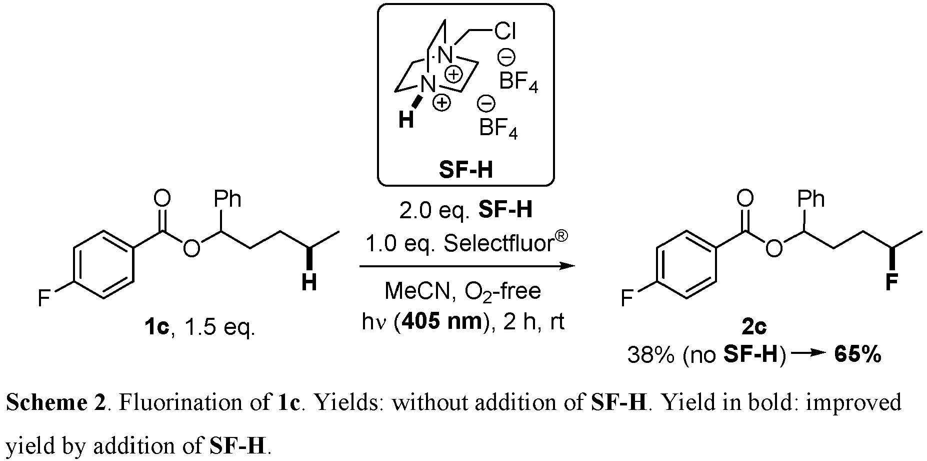

Tan and co-workers developed a photocatalytic energy transfer method for direct fluorination of unactivated C-H bonds employing SF (Selectfluor^{®}) as an electrophilic fluorine source and AQN as a photocatalyst (Kee *et al.,* 2014; Kee *et al.,* 2017). Considering efficiency of our method, we tried to increase the yield of the substrates that gave poor yields. With their standard conditions using 1.0 eq. SF and 2 mol% AQN, we were able to fluorinate methyl (2,2,2-trifluoroacetyl)leucinate (1d) to afford 34% yield of the desired product (2d). When we repeated the experiment with 2.0 eq. of SF-H as an additive, the yield increased to 55% (Scheme 3). Another example of a substrate where our method proved effective was 1,10 - dibromodecane (1e). By adding 2.0 eq. of SF-H as an additive, we increased the yields of fluorinated product from 41% to 62%. Moreover, addition of 2.0 eq. of SF-H as an additive increased the fluorination efficiency for amyl benzoate (1f) from 55% to 81% of the desired fluorinated product (2f).

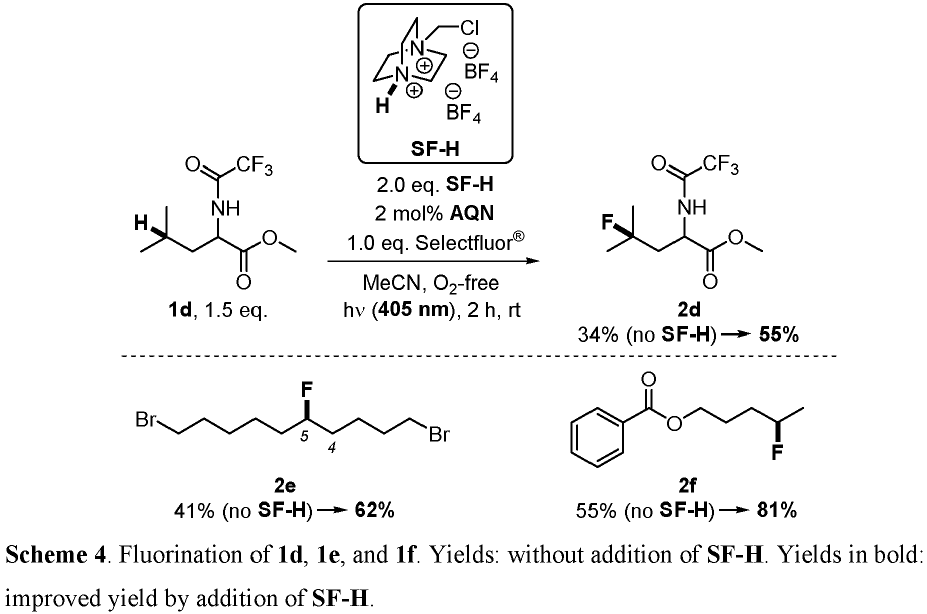

Chen and co-workers discovered acetophenone as a photocatalyst for the direct C-H fluorination of unactivated C(sp³)-H bonds under near-UV light (375-400 nm) (Xia *et al.,* 2013; Xia *et al.,* 2014). The authors could fluorinate hexanone (1g) with 60% yield in 48 h. By attempting to reproduce this data, we achieved a 68% yield of the fluorinated product (2g). However, with 2.0 eq. of SF-H, we were able to increase the yield of 2g from 68% to 96%, once again proving the efficiency of our method (Scheme 4). Moreover, the addition of 2.0 eq. of SF-H increased the yield of fluorinated adamantane from 53% to 74%.

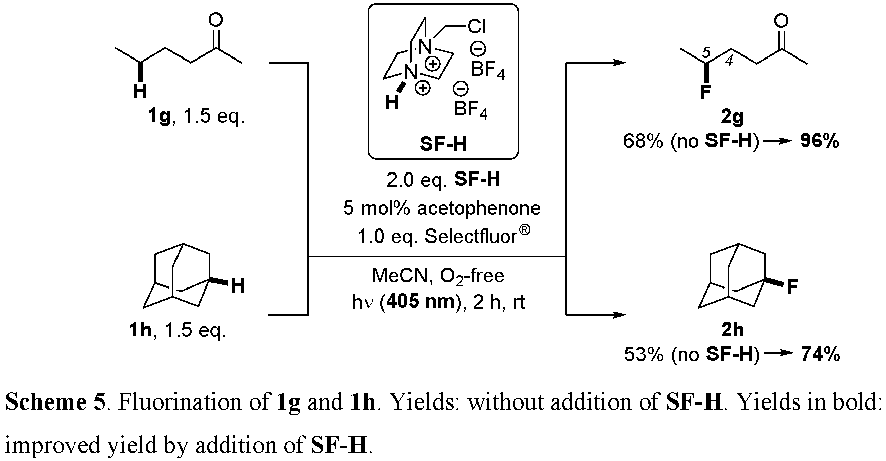

Lectka and co-workers discovered another fluorination method using 1,2,4,5-tetracyanobenzene (TCB) as a photosensitizer under UV-light (Bloom *et al.,* 2014-1; Bloom *et al.,* 2014-2). The authors managed to fluorinate cyclododecane (**1i**) with 63% yield. By attempting to reproduce this data, we achieved a 71% yield of the fluorinated product (2i). However, when running the reaction with addition of 2.0 eq. of SF-H, we were able to increase the yield of 2i from 71% to 94% (Scheme 5).

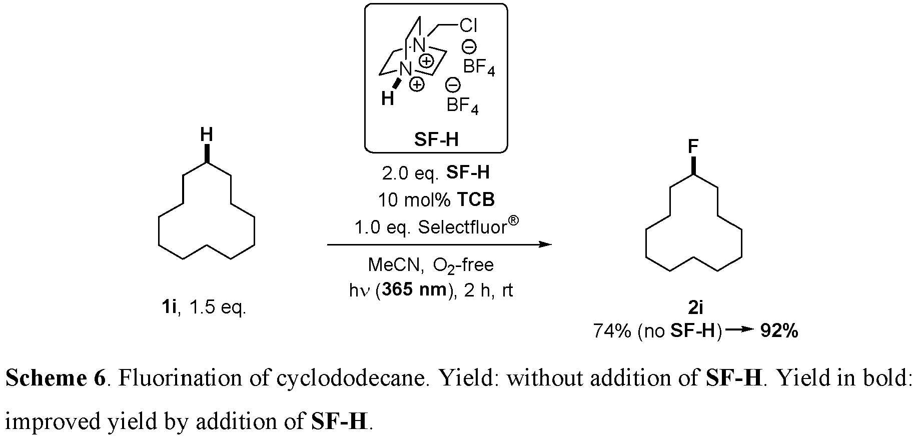

After establishing the effect of SF-H on photochemical fluorinations, our next target was using SF-H in thermal fluorination reactions. Baxter reported a radical C(sp³)-H fluorination method (Hua *et al.,* 2017) using unprotected amino acid - glycine as a radical precursor, catalytic amount of silver nitrate and SF.

With their optimized conditions, they were able to achieve fluorination of a variety of substrates in moderate to excellent yields (30 - 89%). We attempted to fluorinate ibuprofen methyl ester (1a) with the standard conditions, which includes 2.0 eq. of glycine and 2.0 eq. of SF, however, we were only able to achieve 12% of the fluorinated product (2a). Even though we repeated this reaction three times, we could not get higher yields. Gratifyingly, the addition of 2.0 eq. of SF-H to the reaction mixture increased the product yield to 54% (Scheme 6). Another example of a substrate from the same literature that we targeted was 4-methyl acetophenone (1j). In our hands, the standard conditions provided only a 27% yield (upon repeating the experiment three times) of the fluorinated product (2j). However, with the addition of 2.0 eq. of SF-H to the reaction mixture, we were able to increase the yield more than twice - to 60%.

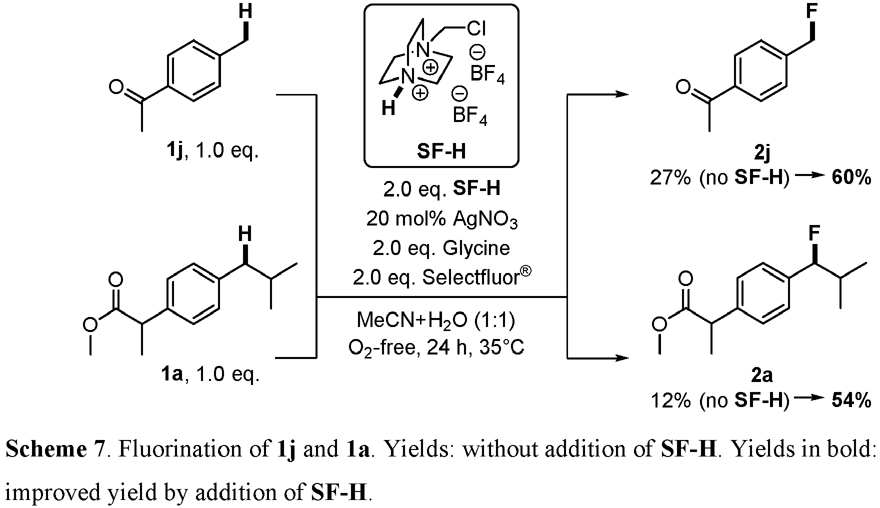

We were able to fluorinate a variety of substrates and achieve higher yields than were reported previously for photochemical reactions (Scheme 7).

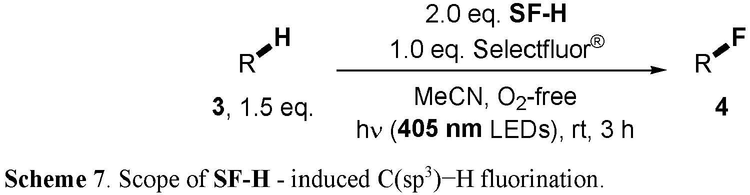

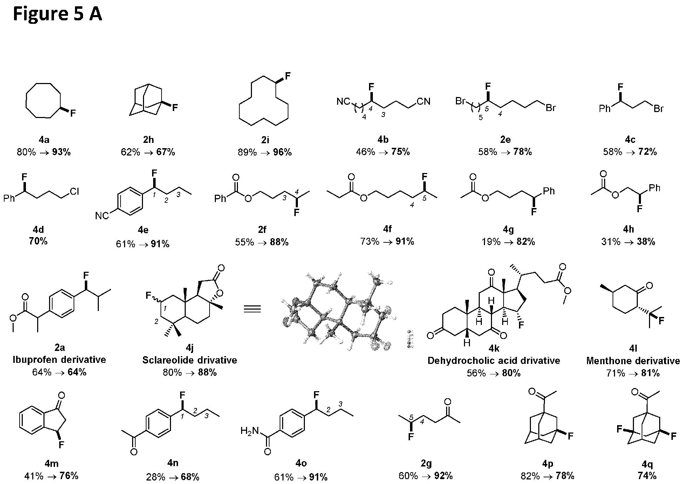

As can be seen also in Figure 5A:
- Secondary and tertiary positions in alkanes were fluorinated (4a, 2h, 2i) in good to excellent yields (67%-96%).
- Terminal halogens were tolerated (2e, 4c, 4d).
- Substrates 4c, 4d, 4g, 4h, 2a, and 4m were fluorinated on benzylic positions and substrates 4e, 4n and 4o provided benzylic fluorinated positions as the major product in good to excellent yields (68%-91%).
- To test the limits of our fluorination method, we applied it to late-stage functionalization of sclareolide (4j) and dehydrocholic acid derivative (4k) that were fluorinated in 88% and 80% yields respectively, which both are higher than previously reported yields (80% and 56% respectively).
- Hexanone was previously fluorinated in a 60% yield, and we were able to achieve a 92% yield of the fluorinated product 2g.

In previous photocatalytic reports, including our auxiliary method (Mei *et al.,* 2019), SF was used as the limiting reagent (1.0 eq. SF and 1.5 eq. of the substrate), and the reactions usually took 24 h. When employing an equimolar amount of SF and the substrate or excess amount of SF, the reaction does not proceed at all or provides only a very low yield of product under the previous literature conditions. A key advantage of the present method (see Scheme 7) is that we can perform the reactions with the substrate as the limiting reagent, which allows us to increase the product yield and to simplify purification (no need to separate the product from the unreacted excess starting material that is normally a challenging endeavor even for fluorinated products). For example, when using SF as the limiting reagent (1.0 eq.) for substrate 4k, a 63% yield was obtained, and when the same substrate was used as the limiting reagent (1.0 eq.), the yield increased to 80% (both reactions in the presence of 2.0 eq SF-H). The same trend was observed for substrates 4b, 4c, 4d, 2f, 4f, 4g, 4j, 4k, and 4m. Although under our conditions herein involving SF-H (2.0 eq) as an additive, substrate 1a (ibuprofen methyl ester) was fluorinated in the same yield as a previously reported photocatalytic method by Lectka (64%) (Bloom *et al.,* 2014-2), in their report the reaction required harsh 302 nm UV light and a 24 h reaction time. Substrate 4p provided slightly lower yield than our previously reported method without SF-H additive (82% to 78%), however, by using substrate as the limiting reagent, we were able to obtain the difluorinated product 4q of substrate 3p in good yield (74%). We demonstrate herein how SF-H grants moderate to good yield improvements in shorter reaction times.

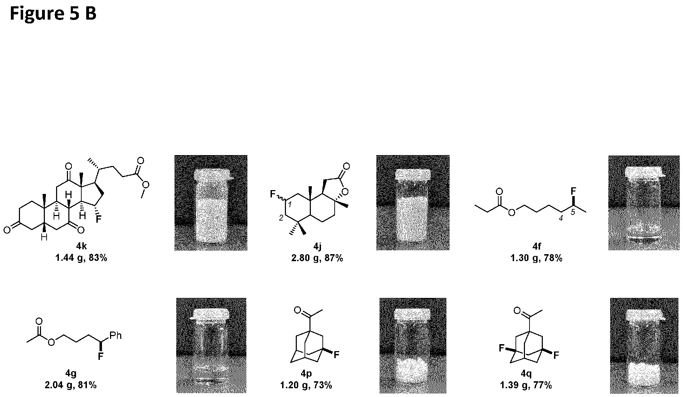

In addition, gram-scale reactions (i.e. 4-12 mmol) of six substrates were performed under 24 h to provide either comparable (4f, 4j, 4g, 4p) or even higher yields (4k, 4q) than their small scale reactions (i.e. 0.2 mmol) (see Figure 5B).

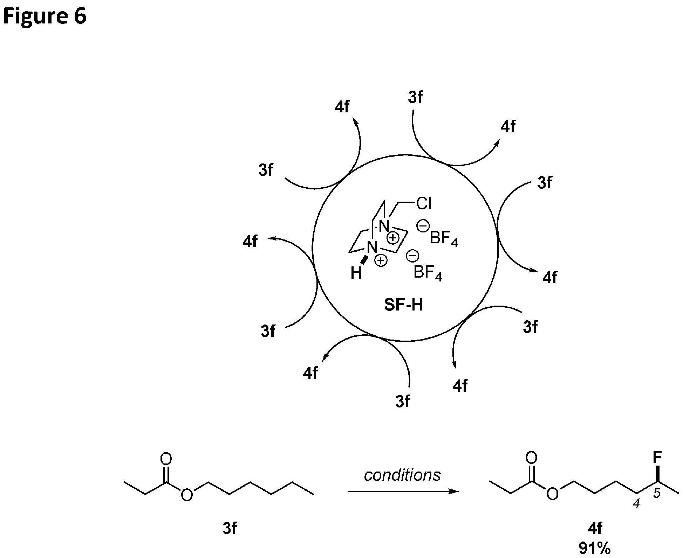

Finally, to demonstrate the surprisingly robust and stable nature of the SF-H, we isolated SF-H after the fluorination reaction of substrate 4f and reused it repeatedly several times, each time isolating it after the reaction and reusing it for the next reaction. The efficiency of SF-H was not diminished at all (see Figure 6).

In conclusion, we report the discovery of a highly efficient, inexpensive additive to increase reaction yield in shorter timeframe for direct various fluorination reactions. Our new additive (SF-H) has been shown to offer an avenue to address some of the constraints in both thermal and photocatalytic fluorination reactions. The yield of some reactions was increased as high as three times, and the duration of some reactions were shortened from 48 h to 2 h. Non-catalytic, SF-H - assisted fluorination was presented that grants moderate to good yield improvements over the previous published methods in shorter reaction times as well as enables gram-scale reactions to be achieved in high yields in under 24 h.

SF-H has the following advantages:
- it is an inexpensive, stable and safe reagent;
- its synthetic preparation and isolation is simple and cheap;
- it can be reused for several reactions;
- it increases the rate of C-H fluorination reactions (efficiency);
- it increase the maximum achievable yield of C-H fluorination reactions.

The following advantages can be achieved with SF-H:
- higher yields;
- shorter reaction times;
- both thermal and photocatalytic fluorinations can be improved;
- it enhances the scalability of fluorination reactions;
- it allows reactions to be done without dry solvents;
- it allows the target substrate to be employed as a limiting reactant;
- it enables photocatalyst-free fluorination reactions.

The following examples and drawings illustrate the present invention without, however, limiting the same thereto.

### BRIEF DESCRIPTION OF THE DRAWINGS

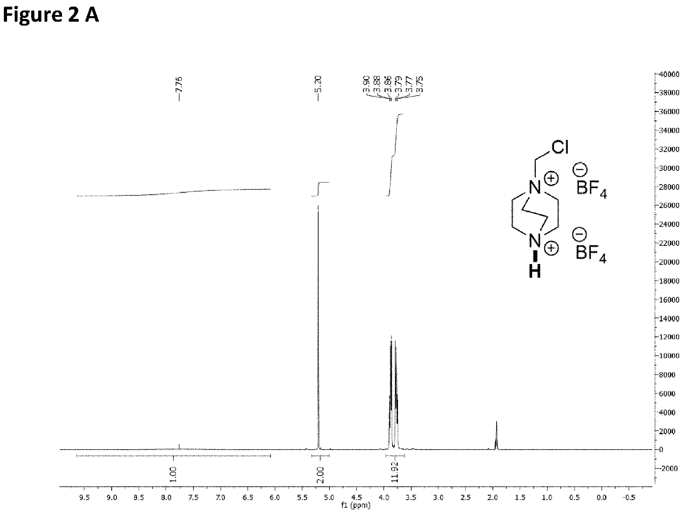

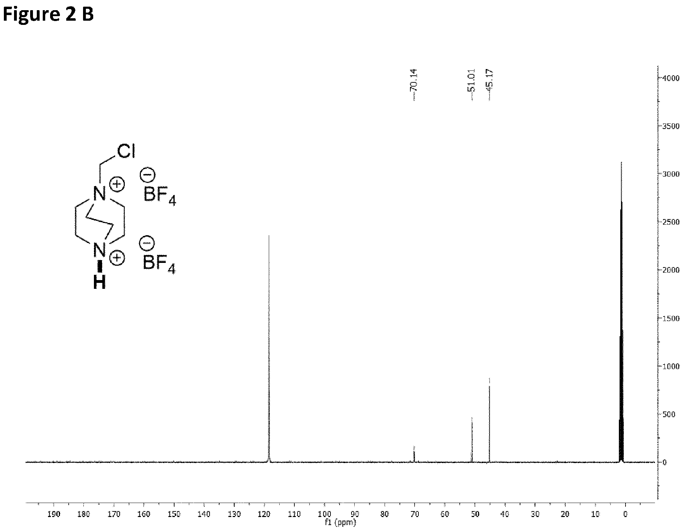

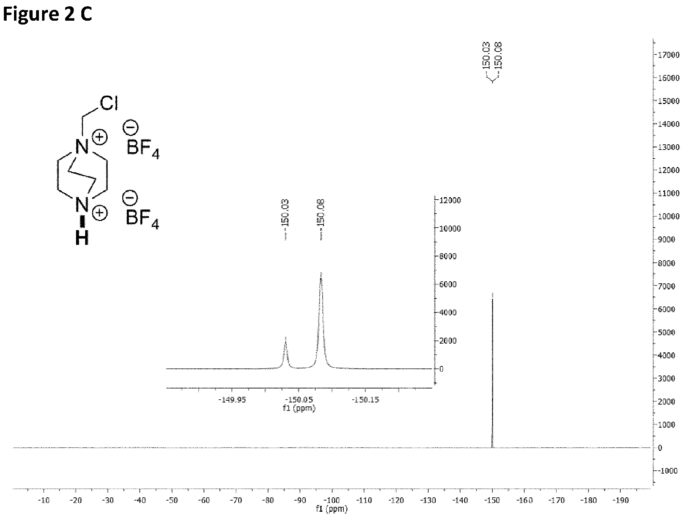

**Figure 1** *Fluorination additive SF-H.*
   **A)** Compound SF-H.
   **B)** Synthesis routes of SF-H. Shown are the three different synthesis pathways A, B and C for compound SF-H-
**Figure 2** *NMR spectra of SF-H.*
   **A)** ¹H NMR of compound SF-H in CD₃CN.
   **B)** ¹³C NMR of compound SF-H in CD₃CN.
   **C)** ¹⁹F NMR of compound SF-H in CD₃CN.

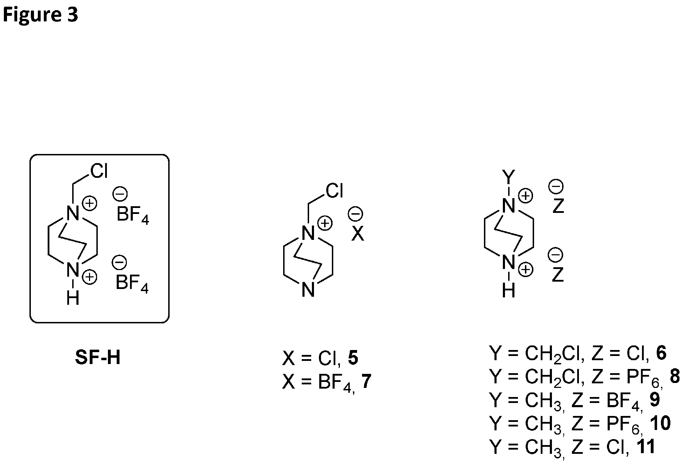

**Figure 3** *Derivatives of SF-H of the present invention.*

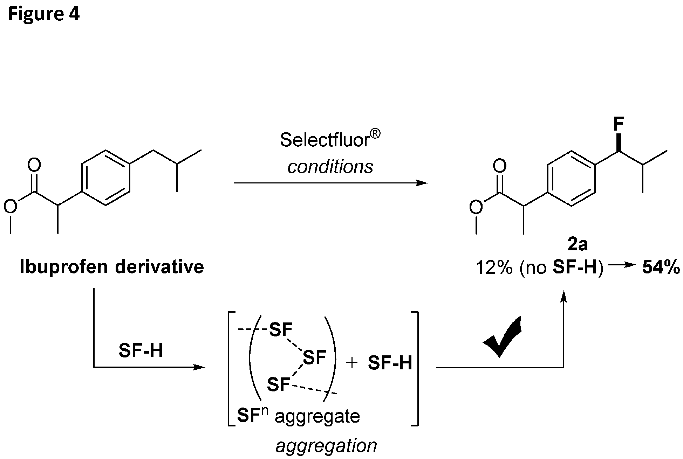

**Figure 4** *Fluorination of ibuprofen with the additive SF-H.*
   The yield could be increased from 12%, as achieved by using SF with the additive SF-H, to about 54%.
**Figure 5** *Scope of SF-H* - *induced C(sp³)-H fluorination.*
   **A)** A variety of different compounds could be fluorinated with the method of the invention.
      Yields shown: previous highest yield reported for catalytic methods. Yields in bold: improved yields by our method using SF-H.
   **B)** Six fluorinated compounds which were generated in gram scale.
**Figure 6** *SF-H can be re-used.*
   Same SF-H was used repeatedly in several fluorination reactions of substrate 3f.

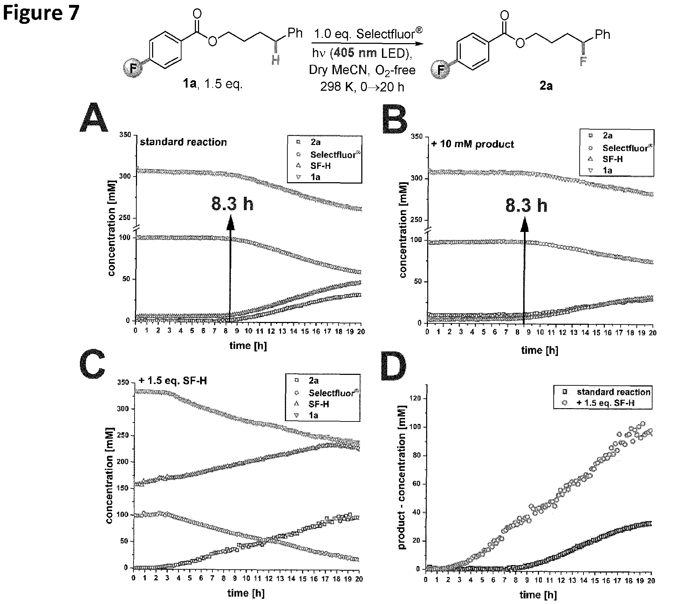

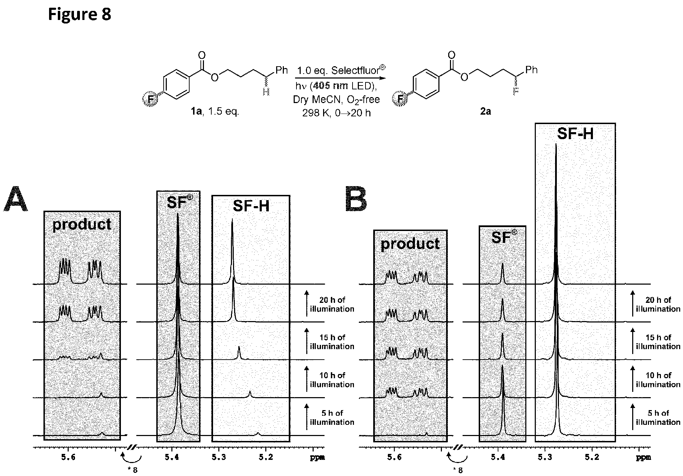

**Figure 7** *¹⁹F{¹H}* in situ *illumination NMR reaction monitoring SF-H effect.*
   Kinetic profiles of the photochemical reaction under standard conditions **(A),** with 10 mM product loading **(B),** and 1.5 eq. SF-H loading **(C).**
   **D)** Detailed comparison of the reaction profiles of product formation without and with 1.5 eq. of SF-H.
**Figure 8**
   **A)** Stacking of ¹H-NMR spectra of photochemical C(sp³)-H fluorination reaction (without SF-H). The spectra were recorded periodically after 5 h of *in situ* illumination.
   **B)** Stacking of ¹H-NMR spectra of photochemical C(sp³)-H fluorination reaction (+ 1.0 eq. of SF-H). The ¹H-NMR spectra were recorded periodically after 5 h *of in situ* illumination.

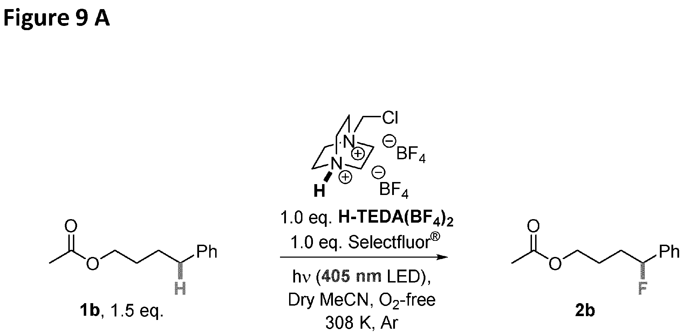

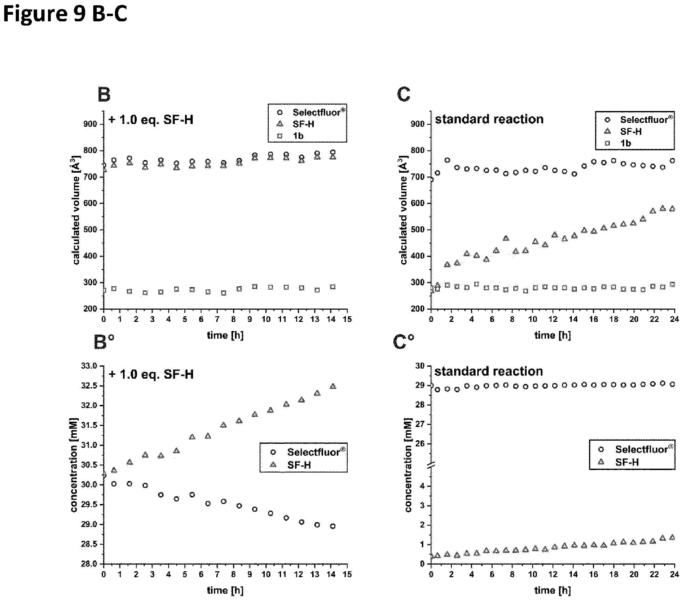

**Figure** 9 *¹**H-NMR* in situ *illumination reaction monitoring of photochemical C(sp³)-H fluorination by* in situ *illumination ID-DSTE DOSY experiments with different SF-H loadings.*
   **A)** Reaction equation of the studied photochemical C(sp³)-H fluorination.
   **B)** and **C)** Calculated volumes [Å³] of components during C(sp³)-H fluorination with 1.0 eq. SF-H loading and without the addition of SF-H (standard reaction). For the size estimation with DOSY, the experimental translational self-diffusion coefficients of the molecules in solution were determined according to the Stej skal-Tanner equation (see the SI for the details).
   (**B**° and **C**°) Change in concentration of SF^{®} and SF-H during *in situ* illumination with 1.0 eq. SF-H loading and without the addition of SF-H (standard reaction).

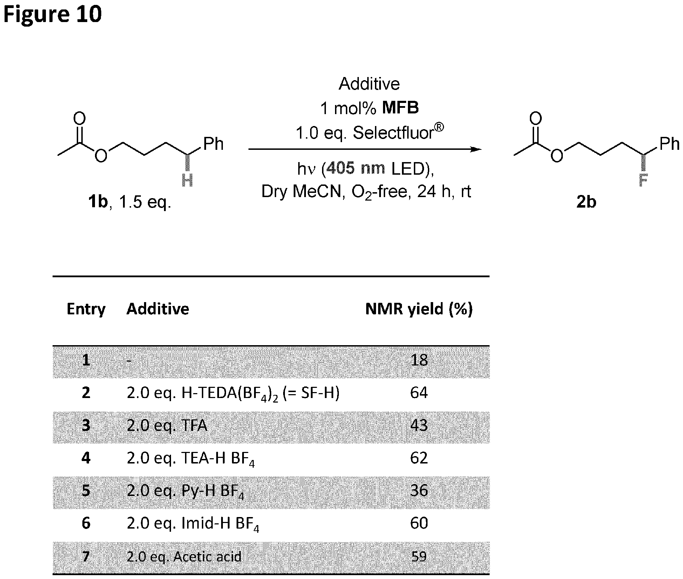

**Figure 10** *Fluorination of 4-phenylbutyl acetate with the SF-H and further additives.* Another substrate which was studied was 4-phenylbutyl acetate (**1b**), which does not contain an attached photosensitizer, such as benzoate and 4-fluorobenzoate, thus it eliminates the possibility of self-fluorination. Substrate **1b** was reported to afford only 18% yield of **2b** when treated with 1 mol% photocatalyst (MFB) under 400 nm irradiation for 24 h (see Table, entry 1). With the addition of 2.0 eq. of SF-H to the reaction mixture, we were able to obtain a 64% yield of the product **2b** (see Table, entry 2). Because the fluorination of **1b** exterminates the possibility of self-fluorination, we examined the effect of other additives in this reaction. Addition of 2.0 eq. TFA in the reaction mixture provided 43% yield, and addition of 2.0 eq. TEA-H BF₄ provided 62% yield (see Table, entries 3 and 4). Other additives, Py-H BF₄, 1H-imidazol-3-ium tetrafluoroborate (Imid-H BF₄), and acetic acid also promoted the reaction to 36%, 60% and 59% yields of **2b** respectively (see Table, entries 5 to 7).

### EXAMPLES

### Example 1

Syntheses of products were confirmed by comparisons with the literature data where possible, HR-MS and by ¹H and ¹⁹F NMR spectra. NMR spectra were recorded in CD₃CN on a Bruker Avance 400 (400 MHz for ¹H, 101 MHz for ¹³C, 376 MHz for ¹⁹F). For ¹H, ¹³C and ¹⁹F chemical shifts are presented in δ-scale as ppm (parts per million) with residual acetonitrile peak as the internal standard (1.96 ppm for 1H and 118.26, 1.79 ppm for 13C). For ¹⁹F NMR, trifluorotoluene was used as the reference (-63.38 ppm), if not stated, no reference is used. NMR yields were calculated based on ¹⁹F NMR using either trifluorotoluene or pentafluorobenzene as an Internal Standard. MestReNova v6.0.2-5475 was used to process NMR spectra. The description of multiplicity that were used is as follows: s = singlet, d = doublet, dd=doublet of doublet, ddd=doublet of doublet of doublet, dm=doublet of multiplet, t = triplet, q = quartet, p=pentet, m = multiplet.

HR-MS were recorded at the Central Analytical Department of the University of Regensburg and the spectra were measured on a JOEL AccuTOF GCx instrument for electron ionization (EI), Agilent Q-TOF 6540 UHD instrument for electrospray ionization (ESI) and atmospheric-pressure chemical ionization (APCI).

### 1.1 Synthesis of SF-H from DABCO

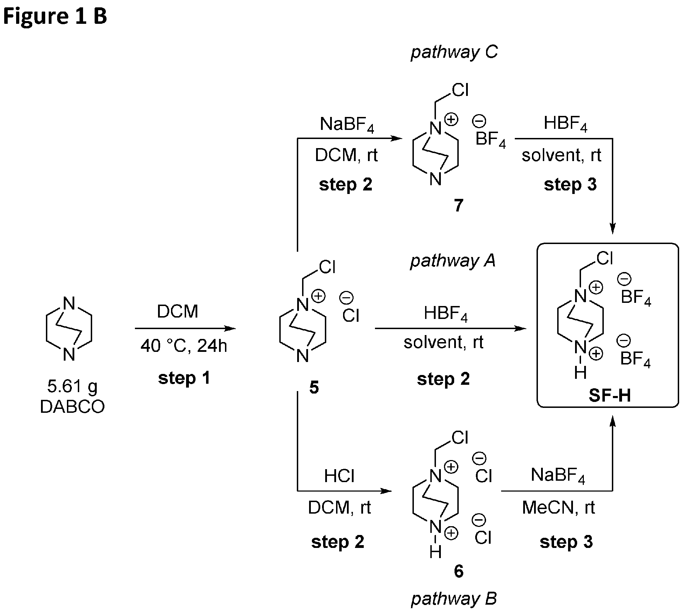

Two different paths for synthesizing SF-H were developed by the inventors: **Pathway A** (including 2 steps) and **Pathway B** (including 3 steps), as shown in Figure 1B.

The first step in both pathways is the same, the alkylation of DABCO.

### Step 1 - alkylation:

1,4-diazabicyclo[2.2.2]octane (DABCO, also known as triethylenediamine (TEDA)) (5.61 g, 50.0 mmol, 1.0 eq.) was dissolved in DCM (100 mL) and refluxed under 40 °C for 24 h.

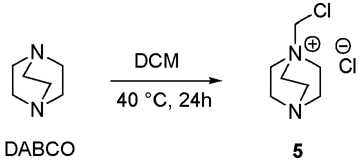

The reaction mixture is further used for Step 2.

### Pathway A:

### Step 2 - counterion exchange and protonation:

To the reaction mixture from *Step 7,* a solution of tetrafluoroboric acid (48 wt.% in H₂O) (20.0 mL, 150 mmol, 3.0 eq.) was added. The reaction mixture was stirred for 1 hour and then was concentrated *in vacuo* to provide the desired product - **SF-H** (quantitative yield, 16.80 g, 49.96 mmol).

Alternatively, the reaction mixture from *Step 1*was concentrated *in vacuo* and the dry product was obtained. The product was dissolved in acetonitrile (50 mL) and a solution of tetrafluoroboric acid (48 wt.% in H₂O) (20.0 mL, 150 mmol, 3.0 eq.) was added to the solution. The reaction mixture was stirred for 1 hour. A white solid was precipitated that was filtered and dried *in vacuo* to provide the desired product - **SF-H** (quantitative yield, 16.81 g, 50.0 mmol).

Alternatively, the reaction mixture from *Step 1* was concentrated *in vacuo* and the dry product was obtained. The product was dissolved in acetone (50 mL) and solution of tetrafluoroboric acid (48 wt.% in H₂O) (20.0 mL, 150 mmol, 3.0 eq.) was added to the solution. The reaction mixture was stirred for 1 hour. A white solid was precipitated that was filtered and dried *in vacuo* to provide the desired product - **SF-H** (quantitative yield, 16.80 g, 49.96 mmol).

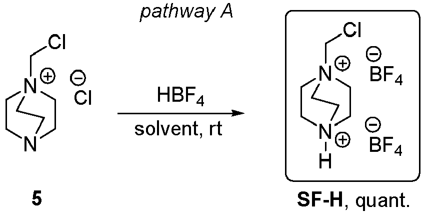

### Pathway B:

### Step 2' - protonation:

To the reaction mixture from *Step 1,* a solution of hydrochloric acid (12 M in H₂O) (8.3 mL, 100 mmol, 2.0 eq.) was added. The reaction mixture was stirred for 1 hour and then was concentrated *in vacuo* to provide white solid as the product (6).

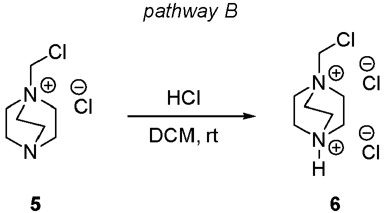

### Step 3' - counterion exchange:

The product **(6)** was added to a solution of sodium tetrafluoroborate (NaBF₄) (11.53 g, 105.0 mmol, 2.1 eq.) in MeCN (100 mL). The reaction mixture was stirred for 1 hour. Sodium chloride was precipitated as a white solid that was filtered out and the filtrate was dried *in vacuo* to provide the desired product - **SF-H** (98% yield, 16,48 g, 49,0 mmol).

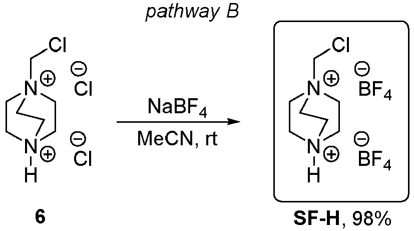

### Pathway C:

### Step 2" - counterion exchange:

To the reaction mixture from *Step 7,* solution of sodium tetrafluoroborate (NaBF₄) (55 mmol, 1.1 eq.) was added. The reaction mixture was stirred for 1 hour and then was concentrated *in vacuo* to provide white solid as the product (7).

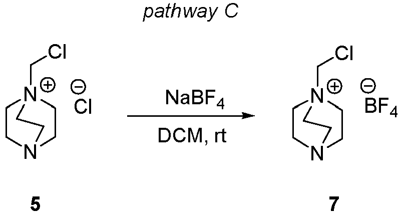

### Step 3" - counterion exchange and protonation

The product from Step **2"** was dissolved in acetonitrile (50 mL) and a solution of tetrafluoroboric acid (48 wt.% in H₂O) (10.0 mL, 75 mmol, 1.5 eq.) was added to the solution. The reaction mixture was stirred for 1 hour. A white solid was precipitated that was filtered and dried *in vacuo* to provide the desired product - **SF-H** (quantitative yield, 16.80 g, 49.96 mmol).

Alternatively, the product from **Step 2"** was dissolved in acetone (50 mL) and a solution of tetrafluoroboric acid (48 wt.% in H₂O) (10.0 mL, 75 mmol, 1.5 eq.) was added to the solution. The reaction mixture was stirred for 1 hour. A white solid was precipitated that was filtered and dried *in vacuo* to provide the desired product - SF-H (quantitative yield, 16.81 g, 50.0 mmol).

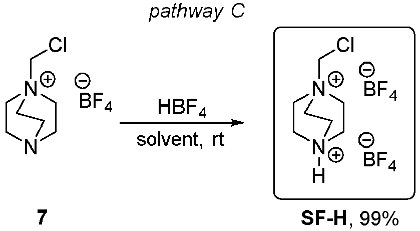

### 1-(chloromethyl)-1,4-diazabicyclo[2.2.2]octane-1,4-diium ditetrafluoroborate (SF-H)

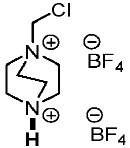

Yield: 97%; white solid; IR (neat) v (cm⁻¹): 3179, 3060, 3012, 1480, 1409, 1338, 1290, 1036, 943, 895, 850, 798, 764, 731; ¹H NMR (400 MHz, CD₃CN) δ 7.45 (brs, 1H), 5.20 (s, 2H), 3.82 (dt, *J=* 15.0, 6.7 Hz, 12H) ppm; ¹³C NMR (101 MHz, CD₃CN) δ 70.1, 51.0, 45.1 ppm; ¹⁹F NMR (377 MHz, CD₃CN) δ -150.0 (s), -150.1 (s) ppm; HRMS (ESI) *(m*/*z)* [M-H]⁺: exact mass calc. for C₇H₁₄ClN₂⁺: 161.0840 found: 161.0837.

### 1.2 Synthesis of SF-H derivatives

Using the synthesis described in 1.1, further compounds **5** to **11** were synthesized:

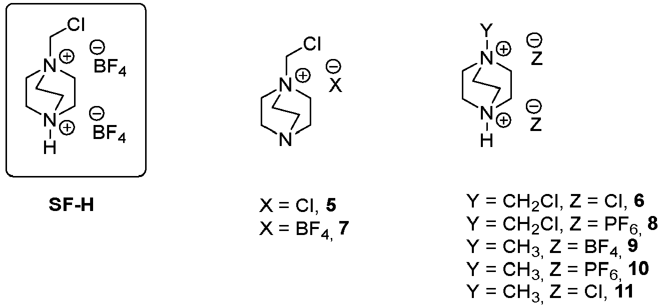

### 1-(chloromethyl)-1,4-diazabicyclo[2.2.2]octan-1-ium chloride (5)

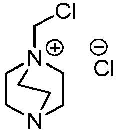

¹H NMR (400 MHz, DMSO) δ 5.67 (s, 2H), 3.62 - 3.50 (m, 6H), 3.18 - 3.01 (m, 6H) ppm; ¹³C NMR (101 MHz, DMSO) δ 68.2, 51.3, 45.3 ppm; HRMS (ESI) *(m*/*z)* [M]⁺: exact mass calc. for C₇H₁₄ClN₂⁺: 161.0840; found: 161.0845.

### 1-(chloromethyl)-1,4-diazabicyclo[2.2.2]octane-1,4-diium dichloride (6)

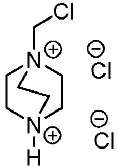

¹H NMR (400 MHz, D₂O) δ 5.48 (s, 2H), 4.19 - 4.07 (m, 6H), 4.03 - 3.91 (m, 6H) ppm; ¹³C NMR (101 MHz, D₂O) δ 69.1, 50.2, 43.9 ppm; HRMS (ESI) *(m*/*z)* [M]⁺: exact mass calc. for C₇H₁₄ClN₂⁺: 161.0840; found: 161.0840.

### 1-(chloromethyl)-1,4-diazabicyclo[2.2.2]octan-1-ium tetrafluoroborate (7)

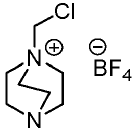

IR (neat) v (cm⁻¹): 3630, 3049, 2974, 2900, 1636, 1469, 1364, 1327, 1290, 1047, 906, 842, 790, 686; ¹H NMR (400 MHz, DMSO) δ 5.29 (s, 2H), 3.48 - 3.35 (m, 6H), 3.20 - 3.06 (m, 6H) ppm; ¹³C NMR (101 MHz, DMSO) δ 68.6, 51.7, 45.4 ppm; ¹⁹F NMR (377 MHz, DMSO) δ -147.9 (s), -148.0 (s) ppm; HRMS (ESI) *(m*/*z)* [M]⁺: exact mass calc. for C₇H₁₄ClN₂⁺: 161.0841 found: 161.0839.

### 1-(chloromethyl)-1,4-diazabicyclo[2.2.2]octane-1,4-diium dihexafluorophosphate (8)

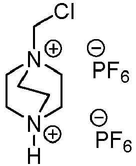

¹H NMR (400 MHz, CD₃CN) δ 6.07 - 5.28 (brs, 1H), 5.16 (s, 2H), 3.82 (m, 6H), 3.71 (m, 6H) ppm; ¹³C NMR (101 MHz, CD₃CN) δ 69.8, 50.7, 44.7 ppm; ¹⁹F NMR (377 MHz, CD₃CN) δ -70.7 (s), -72.6 (s) ppm; ³¹P NMR (162 MHz, CD₃CN) δ -143.1 (hept, *J* = 706.3 Hz) ppm; HRMS (ESI) *(m*/*z)* [M]⁺: exact mass calc. for C₇H₁₄ClN₂⁺: 161.0840; found: 161.0840.

### 1-methyl-1,4-diazabicyclo[2.2.2]octane-1,4-diium ditetrafluoroborate (9)

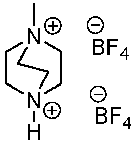

¹H NMR (400 MHz, CD₃CN) δ 6.97 (brs, 1H), 3.73 (dq, *J* = 9.8, 6.2 Hz, 12H), 3.21 (s, 3H) ppm; ¹³C NMR (101 MHz, CD₃CN) δ 53.4 - 53.0 (m), 44.9 (s) ppm; ¹⁹F NMR (377 MHz, CD₃CN) δ -150.0 (s), -150.1 (s) ppm; HRMS (ESI) *(m*/*z)* [M]⁺: exact mass calc. for C₇H₁₅N₂⁺: 127.1230; found: 127.1238.

### 1-methyl-1,4-diazabicyclo[2.2.2]octane-1,4-diium dihexafluorophosphate (10)

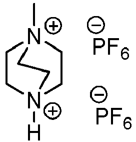

¹H NMR (400 MHz, CD₃CN) δ 7.17 (s, 1H), 3.66 (dq, *J=* 10.4, 6.5 Hz, 12H), 3.18 (s, 3H) ppm; ¹³C NMR (101 MHz, CD₃CN) δ 53.5 - 53.0 (m), 44.7 (s) ppm; ¹⁹F NMR (377 MHz, CD₃CN) δ -70.6 (s), -72.4 (s) ppm; ³¹P NMR (162 MHz, CD₃CN) δ -143.1 (hept, *J* = 706.8 Hz) ppm; HRMS (ESI) *(m*/*z)* [M]⁺: exact mass calc. for C₇H₁₅N₂⁺: 127.1230; found: 127.1232.

### 1-methyl-1,4-diazabicyclo[2.2.2]octane-1,4-diium dichloride (11)

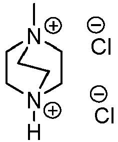

¹H NMR (400 MHz, D₂O) δ 4.08 - 3.87 (m, 12H), 3.40 (s, 3H) ppm, ¹³C NMR (101 MHz, D₂O) δ 53.2 - 52.9 (m), 52.8 - 52.5 (m), 44.0 (s) ppm. HRMS (ESI) *(m*/*z)* [M]⁺: exact mass calc. for C₇H₁₅N₂⁺: 127.1230; found: 127.1230.

### Triethylammonium tetrafluoroborate (TEA-H BF₄)

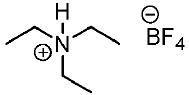

¹H NMR (400 MHz, D₂O) δ 4.79 (s, 1H), 3.27 (q, *J=* 7.3 Hz, 2H), 1.35 (t, *J* = 7.4 Hz, 3H) ppm; ¹³C NMR (101 MHz, D₂O) δ 46.8, 8.3 ppm; ¹⁹F NMR (377 MHz, D₂O) δ -150.3 (s), - 150.4 (s) ppm; HRMS (ESI) *(m*/*z)* [M]⁺: exact mass calc. for C₆H₁₆N⁺: 102.1277; found: 102.1278.

### Pyridin-1-ium tetrafluoroborate (Py-H BF₄)

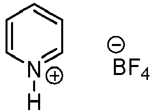

¹H NMR (400 MHz, D₂O) δ 8.79 (d, *J* = 5.3 Hz, 2H), 8.64 (tt, *J* = 7.9, 1.5 Hz, 1H), 8.09 (t, *J* = 7.1 Hz, 2H) ppm; ¹³C NMR (101 MHz, D₂O) δ 147.3, 141.1, 127.5 ppm; ¹⁹F NMR (377 MHz, D₂O) δ -150.4 (s), -150.4 (s) ppm; HRMS (ESI) *(m*/*z)* [M]⁺: exact mass calc. for C₅H₆N⁺: 80.0495; found: 80.0494.

### 1H-imidazol-3-ium tetrafluoroborate (Imid-H BF₄)

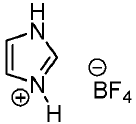

¹H NMR (400 MHz, D₂O) δ 8.72 (s, 1H), 7.52 (s, 2H) ppm; ¹³C NMR (101 MHz, D₂O) δ 133.51, 119.01 ppm; ¹⁹F NMR (377 MHz, D₂O) δ -150.3 (s), -150.4 (s) ppm; HRMS (ESI) *(m*/*z)* [M]⁺: exact mass calc. for C₃H₅N₂⁺: 69.0447; found: 69.0446.

### Benzenaminium tetrafluoroborate (Anyl-H BF₄)

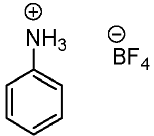

¹H NMR (400 MHz, D₂O) δ 7.54 - 7.44 (m, 3H), 7.41 - 7.35 (m, 2H) ppm; ¹³C NMR (101 MHz, D₂O) δ 130.1, 129.7, 129.3, 122.9 ppm; ¹⁹F NMR (377 MHz, D₂O) δ -150.3 (s), -150.4 (s) ppm; HRMS (ESI) *(m*/*z)* [M]⁺: exact mass calc. for C₆H₈N⁺: 94.0651; found: 94.0651.

### 4-(Dimethylammonio)pyridin-1-ium ditetrafluoroborate (DMAP-H BF₄)

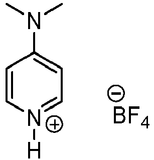

¹H NMR (400 MHz, D₂O) δ 8.00 (d, *J=* 7.7 Hz, 2H), 6.84 (d, *J=* 7.8 Hz, 2H), 3.18 (s, 6H) ppm; ¹³C NMR (101 MHz, D₂O) δ 157.4, 138.2, 106.7, 39.3 ppm; ¹⁹F NMR (377 MHz, D₂O) δ -150.3 (s), -150.4 (s) ppm; HRMS (ESI) *(m*/*z)* [M]⁺: exact mass calc. for C₇H₁₁N₂⁺: 123.0917; found: 123.0917.

### 3-Benzyl-1-methyl-1H-imidazol-3-ium hexafluorophosphate (BnMIM-H PF₆)

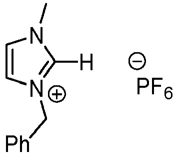

¹H NMR (400 MHz, DMSO) δ 9.23 (s, 1H), 7.79 (t, *J=* 1.7 Hz, 1H), 7.71 (t, *J=* 1.7 Hz, 1H), 7.55 - 7.30 (m, 5H), 3.91 (s, 3H) ppm; ¹³C NMR (101 MHz, DMSO) δ 137.7, 135.7, 130.0, 129.8, 129.3, 125.0, 123.3, 52.9, 36.8 ppm; ¹⁹F NMR (377 MHz, DMSO) δ -68.7 (s), -70.6 (s) ppm; ³¹P NMR (162 MHz, DMSO) δ -128.8 - -157.0 (m) ppm; HRMS (ESI) *(m*/*z)* [M]⁺: exact mass calc. for C₁₁H₁₃N₂⁺: 173.1073; found: 173.1073.

The features disclosed in the foregoing description, in the claims and/or in the accompanying drawings may, both separately and in any combination thereof, be material for realizing the invention in diverse forms thereof.

### REFERENCES

A. Albini, M. Fagnoni, Green Chem. 2004, 6, 1.
A. Albini, M. Fagnoni, ChemSusChem 2008, 1, 63.
Y. Amaoka, M. Nagatomo, M. Inoue, Org. Lett. 2013, 15, 2160.
S. Bloom, J. L. Knippel, T. Lectka, Chem. Sci. 2014, 5, 1175.
S. Bloom, M. McCann, T. Lectka, Org. Lett. 2014, 16, 6338.
D. D. Bume, S. A. Harry, T. Lectka, C. R. Pitts, J. Org. Chem. 2018, 83, 8803.
H. Egami, S. Masuda, Y. Kawato, Y. Hamashima, Org. Lett. 2018, 20, 1367.
W. K. Hagmann, J. Med. Chem. 2008, 51, 4359.
A. M. Hua, D. N. Mai, R. Martinez, R. D. Baxter, Org. Lett. 2017, 19, 2949.
C. W. Kee, K. F. Chin, M. W. Wong, C. H. Tan, Chem. Commun. 2014, 50, 8211.
J. W. Kee, H. Shao, C. W. Kee, Y. Lu, H. S. Soo, C. H. Tan, Catal. Sci. Technol. 2017, 7, 848.
B. Lantaño, A. Postigo, Org. Biomol. Chem. 2017, 15, 9954
T. Liang, C. N. Neumann, T. Ritter, Angew. Chem., Int. Ed. 2013, 52, 8214
P. Maienfisch, R. G. Hall, Chimia 2004, 58, 93.
H. Mei, J. Han, S. Fustero, M. Medio-Simon, D. M. Sedgwick, C. Santi, R. Ruzziconi, V. A. Soloshonok, Chem. - Eur. J. 2019, 25, 11797.
K. Müller, C. Faeh, F. Diederich, Science 2007, 317, 1881.
S. Purser, P. R. Moore, S. Swallow, V. Gouverneur, Chem. Soc. Rev. 2008, 37, 320.
H. D. Roth, Angew. Chem., Int. Ed. Engl. 1989, 28, 1193.
G. Theodoridis, Advances in Fluorine Science; Elsevier: Amsterdam, Netherlands, 2006; Vol. 2, pp 121-175.
J. B. Xia, C. Zhu, C. Chen, J. Am. Chem. Soc. 2013, 135, 17494.
J. B. Xia, C. Zhu, C. Chen, Chem. Commun. 2014, 50, 11701.
S. Yakubov, J. P. Barham, Beilstein J. Org. Chem. 2020, 16, 2151.
S. Yakubov, W. J. Stockerl, X. Tian, A. Shahin, M. J. P. Mandigma, R. M. Gschwind, J. P. Barham, Chem. Sci., 2022, 13, 14041.

## Claims

1. Use of 1-(chloromethyl)-1,4-diazabicyclo[2.2.2]octane-1,4-diium ditetrafluoroborate (SF-H) as additive in a C-H fluorination reaction of a compound with a fluorination agent.

2. The use of claim 1, wherein the fluorination agent is an organic fluorination agent which is preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetrafluoroborate) (SF II) and *N*-Fluorobenzenesulfonimide (NFSI).

3. Use of a hydrogen bond donor compound as additive in a C-H fluorination reaction of a compound with a fluorination agent,
wherein the fluorination agent is preferably an organic fluorination agent which is more preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetraetrafluoroborate) (SF II) and *N*-Fluorobenzenesulfonimide (NFSI).

4. The use of claim 3, wherein the hydrogen bond donor compound is a Bronsted acid, which preferably has a pKa of less than about 15 in water or DMSO,
which is more preferably selected from an organic acid having a formula selected from formula I, II, III, IVa or IVb: 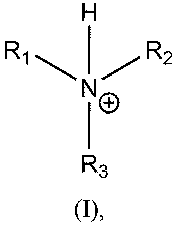 wherein
R¹, R² and R³ are each independently selected from
H,
an unsubstituted or substituted aliphatic carbon chain (such as C₁-C₆ alkyl, C₁-C₆ alkenyl, C₁-C₆ alkynyl),
C₃-C₁₀ heterocyclyl,
unsubstituted or substituted aromatic carbon chain (such as C₃-C₁₀ aryl), and halogen, and
can be the same or different, wherein not all of R¹, R² and R³ are H; 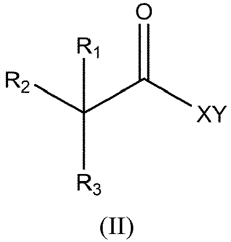
wherein
R¹, R² and R³ are each independently selected from
H,
an unsubstituted or substituted aliphatic carbon chain (such as C₁-C₆ alkyl, C₁-C₆ alkenyl, C₁-C₆ alkynyl),
C₃-C₁₀ heterocyclyl,
unsubstituted or substituted aromatic carbon chain (such as C₃-C₁₀ aryl), and halogen, and
can be the same or different,
X is S or O,
Y is H(¹H) or ²H; 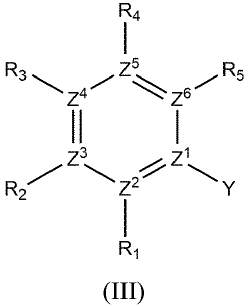
wherein
R¹ to R⁵ are each independently selected from
H,
an unsubstituted or substituted aliphatic carbon chain (such as C₁-C₆ alkyl, C₁-C₆ alkenyl, C₁-C₆ alkynyl),
C₃-C₁₀ heterocyclyl,
unsubstituted or substituted aromatic carbon chain (such as C₃-C₁₀ aryl), and -NR⁶,
halogen, and
can be the same or different, and,
R⁶ is H or C₁-C₆ alkyl,
Y is H(¹H) or ²H,
Z¹ to Z⁶ are each independently selected from C, N, O and S,
wherein not all of Z¹ to Z⁶ are C, or
wherein not all of Z¹ to Z⁶ are N, or
wherein not all of Z¹ to Z⁶ are O or
wherein not all of Z¹ to Z⁶ are S; 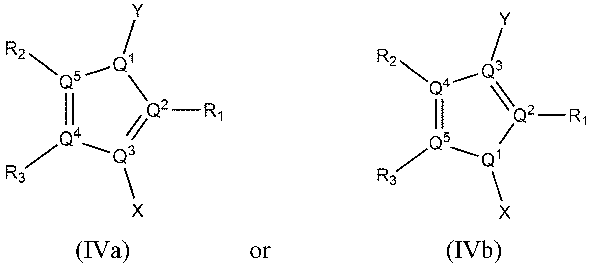
wherein
R¹, R² and R³ are each independently selected from
H,
an unsubstituted or substituted aliphatic carbon chain (such as C₁-C₆ alkyl, C₁-C₆ alkenyl, C₁-C₆ alkynyl),
C₃-C₁₀ heterocyclyl,
unsubstituted or substituted aromatic carbon chain (such as C₃-C₁₀ aryl), and
halogen, and
can be the same or different,
X is selected from H(¹H), ²H or R⁴,
Y is selected from H(¹H), ²H or R⁴,
R⁴ is selected from an unsubstituted or substituted aliphatic carbon chain (such as C₁-C₆ alkyl, C₁-C₆ alkenyl, C₁-C₆ alkynyl), C₃-C₁₀ heterocyclyl, unsubstituted or substituted aromatic carbon chain (such as C₃-C₁₀ aryl), and halogen,
Q¹ to Q⁵ are each independently selected from C, N, O and S,
wherein not all of Q¹ to Q⁵ are C, or
wherein not all of Q¹ to Q⁵ are N, or
wherein not all of Q¹ to Q⁵ are O or
wherein not all of Q¹ to Q⁵ are S;
such as 1-(chloromethyl)-1,4-diazabicyclo[2.2.2]octane-1,4-diium ditetrafluoroborate (SF-H).

5. The use of any one of the preceding claims, wherein the C-H fluorination reaction is an electrophilic, a photochemical, a thermal, a photocatalytic and/or a photosensitized fluorination reaction
and/or wherein the hydrogen bond donor compound is used in a ratio of about 1:20 to about 20:1 of the fluorination agent, preferably about 1:10 to about 10:1, wherein the fluorination agent is preferably SF,
wherein preferably the hydrogen bond donor compound is used in excess of the fluorination agent, preferably SF,
wherein preferably the excess of hydrogen bond donor compound is in the range from about 10 to about 1.5,
and/or wherein hydrogen bond donor compound, preferably SF-H, is reusable.

6. The use of any one of the preceding claims, wherein the compounds to be fluorinated are bioactive molecules, such as pharmaceuticals and/or agrochemicals,
and/or wherein the fluorinated compounds can be obtained in gram scale.

7. A method for the C-H fluorination of a compound with a fluorinating agent, comprising the step:
adding 1-(chloromethyl)-1,4-diazabicyclo[2.2.2]octane-1,4-diium ditetrafluoroborate (SF-H) as additive,
wherein the fluorination agent is preferably an organic fluorination agent which is more preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetraetrafluoroborate) (SF II) and N-Fluorobenzenesulfonimide (NFSI).

8. A method for the C-H fluorination of a compound with a fluorination agent, comprising the step:
adding a hydrogen bond donor compound as additive,
wherein the fluorination agent is preferably an organic fluorination agent which is more preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetraetrafluoroborate) (SF II) and N-Fluorobenzenesulfonimide (NFSI),
wherein the hydrogen bond donor compound is preferably a Bronsted acid, which preferably has a pKa of less than about 15 in water or DMSO,
which is more preferably selected from an organic acid having a formula selected from formula I, II, III, IVa or IVb: 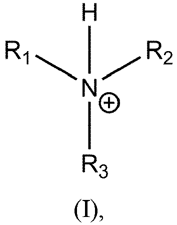 wherein
R¹, R² and R³ are each independently selected from
H,
an unsubstituted or substituted aliphatic carbon chain (such as C₁-C₆ alkyl, C₁-C₆ alkenyl, C₁-C₆ alkynyl),
C₃-C₁₀ heterocyclyl,
unsubstituted or substituted aromatic carbon chain (such as C₃-C₁₀ aryl), and halogen, and
can be the same or different, wherein not all of R¹, R² and R³ are H; 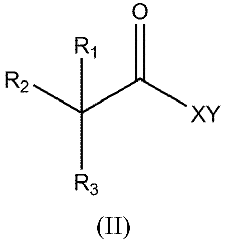
wherein
R¹, R² and R³ are each independently selected from
H,
an unsubstituted or substituted aliphatic carbon chain (such as C₁-C₆ alkyl, C₁-C₆ alkenyl, C₁-C₆ alkynyl),
C₃-C₁₀ heterocyclyl,
unsubstituted or substituted aromatic carbon chain (such as C₃-C₁₀ aryl), and
halogen, and
can be the same or different,
X is S or O,
Y is H(¹H) or ²H; 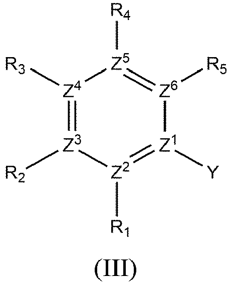
wherein
R¹ to R⁵ are each independently selected from
H,
an unsubstituted or substituted aliphatic carbon chain (such as C₁-C₆ alkyl, C₁-C₆ alkenyl, C₁-C₆ alkynyl),
C₃-C₁₀ heterocyclyl,
unsubstituted or substituted aromatic carbon chain (such as C₃-C₁₀ aryl), and -NR⁶,
halogen, and
can be the same or different, and,
R⁶ is H or C₁-C₆ alkyl,
Y is H(¹H) or ²H,
Z¹ to Z⁶ are each independently selected from C, N, O and S,
wherein not all of Z¹ to Z⁶ are C, or
wherein not all of Z¹ to Z⁶ are N, or
wherein not all of Z¹ to Z⁶ are O or
wherein not all of Z¹ to Z⁶ are S; 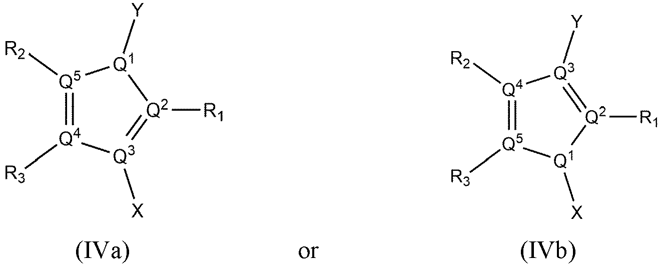
wherein
R¹, R² and R³ are each independently selected from
H,
an unsubstituted or substituted aliphatic carbon chain (such as C₁-C₆ alkyl, C₁-C₆ alkenyl, C₁-C₆ alkynyl),
C₃-C₁₀ heterocyclyl,
unsubstituted or substituted aromatic carbon chain (such as C₃-C₁₀ aryl), and halogen, and
can be the same or different,
X is selected from H(¹H), ²H or R⁴,
Y is selected from H(¹H), ²H or R⁴,
R⁴ is selected from an unsubstituted or substituted aliphatic carbon chain (such as C₁-C₆ alkyl, C₁-C₆ alkenyl, C₁-C₆ alkynyl), C₃-C₁₀ heterocyclyl, unsubstituted or substituted aromatic carbon chain (such as C₃-C₁₀ aryl), and halogen,
Q¹ to Q⁵ are each independently selected from C, N, O and S,
wherein not all of Q¹ to Q⁵ are C, or
wherein not all of Q¹ to Q⁵ are N, or
wherein not all of Q¹ to Q⁵ are O or
wherein not all of Q¹ to Q⁵ are S;
such as 1-(chloromethyl)-1,4-diazabicyclo[2.2.2]octane-1,4-diium ditetrafluoroborate (SF-H).

9. The method of claim 7 or 8, wherein the C-H fluorination reaction is an electrophilic, a photochemical, a thermal, a photocatalytic and/or a photosensitized fluorination reaction;
and/or wherein the hydrogen bond donor compound is used in a ratio of about 1:20 to about 20:1 of the fluorination agent, more preferably about 1:10 to about 10:1, wherein the fluorination agent is preferably SF,
wherein preferably is used in excess of the fluorination agent, preferably SF,
wherein preferably the excess of the hydrogen bond donor compound is in the range from about 10 to about 1.5;
and/or wherein the compounds to be fluorinated are bioactive molecules, such as pharmaceuticals and/or agrochemicals,
and/or wherein the fluorinated compounds can be obtained in gram scale;
and/or wherein the hydrogen bond donor compound, more preferably SF-H, is reusable.

10. A method of synthesizing 1-(chloromethyl)-1,4-diazabicyclo[2.2.2]octane-1,4-diium ditetrafluoroborate (SF-H), comprising the steps of
1) alkylation of 1,4-diazabicyclo[2.2.2]octane in dichloromethane;
2) protonation and ion exchange via addition of tetrafluoroboric acid to the mixture of step 1) and obtaining SF-H;
or
2') protonation via addition of hydrochloric acid to the mixture of step 1) and obtaining 1-(chloromethyl)-1,4-diazabicyclo[2.2.2]octane-1,4-diium dichloride;
3') ion exchange via addition of a solution of sodium tetrafluoroborate (NaBF₄) to the product of step 2') and obtaining SF-H;
or
2") ion exchange via addition of a solution of NaBF₄ to the product of step 1) and obtaining 1-(chloromethyl)-1,4-diazabicyclo[2.2.2]octan-1-ium tetrafluoroborate;
3") protonation and ion exchange via addition of tetrafluoroboric acid to the product of step 2") and obtaining SF-H.

11. The method of claim 10, wherein in Step 2) before the tetrafluoroboric acid is added, the reaction mixture of Step 1) is concentrated and a dry product is obtained, which is then dissolved in a solvent, such as acetonitrile or acetone.

12. A compound having the formula V or VI 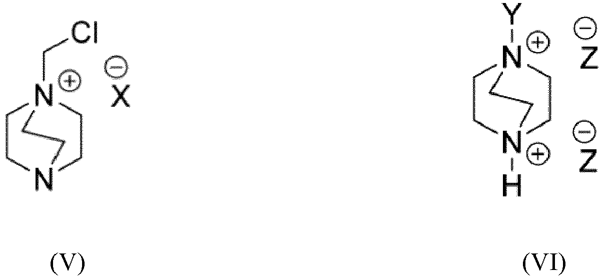 wherein
**X** is Cl or BF₄,
**Y** is CH₂Cl or CH₃, and
**Z** is Cl, BF₄ or PF₆;
wherein, preferably the compound has formula V and
**X** is Cl or
**X** is BF₄,
or the compound has formula VI and
**Y** is CH₂Cl and **Z** is BF₄, or
**Y** is CH₂Cl and **Z** is Cl, or
**Y** is CH₂Cl and **Z** is PF₆, or
**Y** is CH₃ and **Z** is BF₄, or
**Y** is CH₃ and **Z** is Cl, or
**Y** is CH₃ and **Z** is PF₆.

13. A compound selected from

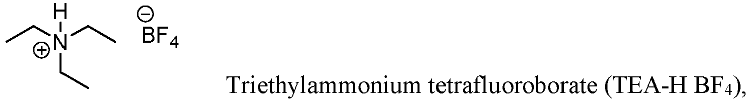

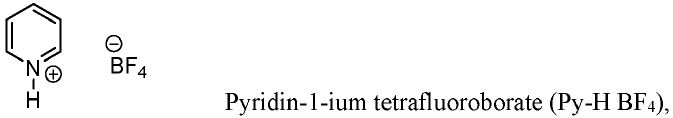

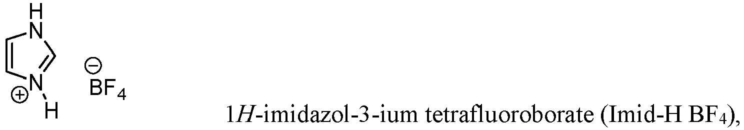

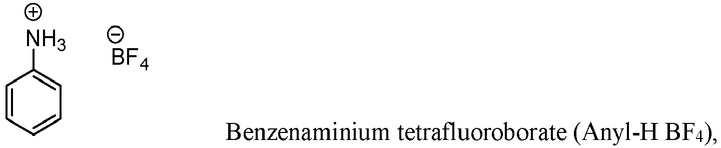

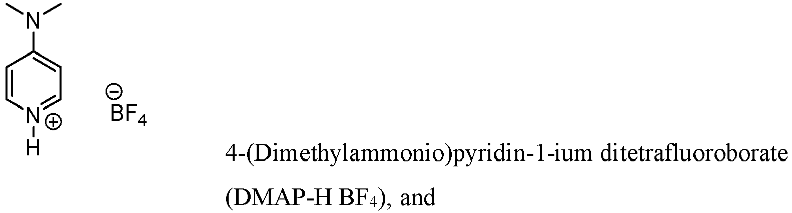

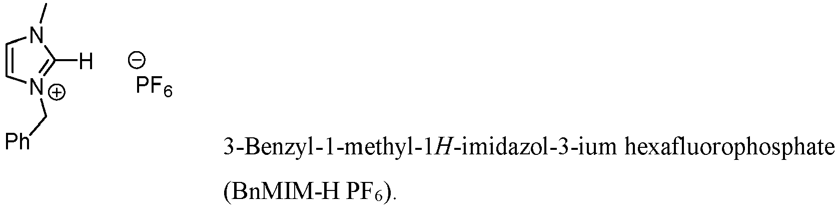

14. Use of a compound of claim 12 or 13 as additive in a C-H fluorination reaction of a compound with a fluorination agent,
wherein the fluorination agent is preferably an organic fluorination agent, which is more preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetraetrafluoroborate) (SF II) and *N*-Fluorobenzenesulfonimide (NFSI),
and/or wherein the C-H fluorination reaction is preferably an electrophilic, a photochemical, a thermal, a photocatalytic and/or a photosensitized fluorination reaction,
and/or wherein the compounds to be fluorinated are preferably bioactive molecules, such as pharmaceuticals and/or agrochemicals,
and/or wherein the fluorinated compounds can preferably be obtained in gram scale.

15. Use of a hydrogen bond donor compound as additive in a C-H fluorination reaction of a compound with a fluorination agent,
wherein the fluorination agent is preferably an organic fluorination agent which is more preferably selected from 1-(chloromethyl)-4-fluoro-1,4-diazabicyclo[2.2.2]octane-1,4-diium ditetrafluoroborate (SF), 1-Fluoro-4-methyl-1,4-diazoniabicyclo[2.2.2]octane-bis-(tetraetrafluoroborate) (SF II) and *N*-Fluorobenzenesulfonimide (NFSI),
wherein the hydrogen bond donor compound is preferably a compound of claim 12 or 13,
and/or wherein the C-H fluorination reaction is preferably an electrophilic, a photochemical, a thermal, a photocatalytic and/or a photosensitized fluorination reaction,
and/or wherein the compounds to be fluorinated are preferably bioactive molecules, such as pharmaceuticals and/or agrochemicals,
and/or wherein the fluorinated compounds can preferably be obtained in gram scale.
